# EUROPEAN PATENT APPLICATION

(11) **EP 4 692 365 A1**
(43) Date of publication of application: **11.02.2026**
(21) Application number: 24193609.5
(22) Date of filing: 08.08.2024
(51) Int. Cl.: C12P 19/60, C12N 9/10

(54) **GENES, PROTEINS, AND PROCESSES FOR THE SYNTHESIS OF BLUE ANTHOCYANINS IN EUKARYOTIC CELLS**

(71) Applicant: Leibniz-Institut für Pflanzenbiochemie (IPB) Stiftung des öffentlichen Rechts, 06120 Halle (Saale) (DE)
(72) Inventor: MARILLONNET, Sylvestre, 06120 Halle (Saale) (DE)
(74) Representative: Wächtershäuser & Hartz Patentanwaltspartnerschaft mbB

(57) **Abstract**

The invention provides a process of producing delphinidin 3-O-[2-O-(6-O-p-coumaroyl)-glucosyl-6-O-p-coumaroyl] glucoside from delphinidin 3-O-(2-O-glucosyl-6-O-p-coumaroyl) glucoside. The invention also provides processes for the production of delphinidin 3-O-[2-O-(6-O-p-coumaroyl-glucosyl)-6-O-p-coumaroyl] glucoside 5-O-(6-O-malonyl)-glucoside. Enzymes allowing production of these compounds and genes encoding the enzymes are also provided.

## Description

### FIELD OF THE INVENTION

The invention provides a process of producing the delphinidin derivative (IV) comprising the cation of formula (**IV**) or a neutral form or anionic form thereof from the delphinidin derivative (III) comprising the cation of formula (**III**) or a neutral form or anionic form thereof. The invention also provides a process of producing the delphinidin derivative (V) comprising the cation of formula (**V**) or a neutral form or anionic form thereof from the delphinidin derivative (IV) comprising the cation of formula (**IV**) or a neutral form or anionic form thereof. The also invention provides a process of producing the delphinidin derivative (III) comprising the cation of formula (**III**) or a neutral form or anionic form thereof from the delphinidin derivative (II) comprising the cation of formula (**II**) or a neutral form or anionic form thereof. The invention further provides a process of producing the delphinidin derivative (II) comprising the cation of formula (**II**) or a neutral form or anionic form thereof from the delphinidin derivative (I) comprising the cation of formula (**I**) or a neutral form or anionic form thereof. Moreover, the invention provides a process of producing the delphinidin derivative (VI) comprising the cation of formula (**VI**) or a neutral form or anionic form thereof from the delphinidin derivative (V) comprising the cation of formula (V) or a neutral form or anionic form thereof.

The invention also relates to proteins capable of catalyzing the above reactions and to polynucleotides encoding these proteins. The invention further relates to vectors and constructs comprising one or more of said polynucleotides and to a plant or plant cell transfected or transformed with such polynucleotide, construct or vector.

### BACKGROUND OF THE INVENTION

Blue pigments are rare in nature. In plants, less than 10% of angiosperms are thought to include varieties with blue flowers [5]. As a result, many cultivated plants species do not have cultivars producing blue flowers. And despite years of work by plant breeders, blue roses, tulips, and orchids are still unavailable, as are blue flower cultivars for many other commercially grown flowers. In response to the lack of natural blue flowers in many commercially important species, companies have resorted to producing artificially stained blue flowers with synthetic dyes. This is achieved by either placing cut flowers in water with a blue dye or injecting a blue dye into the stem. However, artificially colored plants do not have a natural appearance and lack stable coloration, as new emerging flowers will typically grow white. Therefore, there is a need for producing flowers with a wider range of natural blue hues, and without the need for artificially staining the flowers.

Blue pigments are also important in the food industry. In Europe, only three synthetic blue pigments are allowed as food colorant: Patent blue V, Brilliant blue FCF and indigo carmine [5,6]. Although these synthetic pigments provide a stable blue color in food preparations at various pH levels, there is growing consumer preference for avoiding synthetic pigments in food. Only one natural pigment, from cyanobacteria (spirulina), is available, but it has a slightly greenish hue. Thus, the food industry needs additional natural blue colorants with different hues. There are currently no blue pigments derived from plants that can be utilized on a large scale as food colorant. This is because blue pigments from plants are only found in flowers, making their production unsuitable for scale up. Anthocyanin pigments can be found in larger amounts in fruits such as cherries, blackcurrant and blueberries. However, despite what the name of blueberries suggests, anthocyanins present in fruits have only red to purple colors. Therefore, there is a need for development of systems for production of natural blue pigments on a large scale. This could consist of production of blue anthocyanins in plants tissues that can be harvested at a large scale, such as for example tomato fruits. Another possibility would be production of anthocyanins in microorganisms such as yeast.

Several plants have blue flowers. Examples include blue gentians such as *Gentiana scabra and Gentiana verna, Delphinium grandiflorum, Centaurea cyanus, Commelina communis, Clitoria ternatea, Salvia patens and Ipomea purpurea.* Many studies have investigated the mechanisms by which blue color is produced in plants. The presence of an anthocyanin component is essential, but often, cofactors such as flavones and metals ions are required for obtaining the blue color [5]. While the chemical structures of the anthocyanins of many blue species have been well characterized, there is still limited knowledge on the genes involved in the biosynthetic pathways that lead to biosynthesis of blue anthocyanins [7]. At present, genes for the complete biosynthetic pathway of a blue anthocyanin have been identified for only one species, *Gentiana triflora* [8-10]. This knowledge should enable engineering of blue flowers in species that do not naturally produce blue anthocyanins. However, the blue anthocyanin from *Gentiana triflora,* gentiodelphin, contains caffeoyl residues, for which acyl donor precursors are not necessarily available in all plants, making reconstitution of the entire biosynthetic pathway in heterologous hosts challenging. The anthocyanidin delphinidin, like nearly all other anthocyanidins, is pH-sensitive, and changes from blue in slightly basic solution to purple and red in more acidic solution over multiple protonation/deprotonation steps (for more detail, see Houghton et al., Plants 2021, 10, 726; doi.org/10.3390/plants10040726).

For several other species with blue flowers, some, but not all genes of the pathways have been identified. This is the case for the biosynthetic pathway of the blue anthocyanin of butterfly pea *(Clitoria ternatea*), for which one glucosyltransferase has been identified [11]. This enzyme was heterologously expressed in chrysanthemum, leading to production of the first genetically engineered blue flowers [11]. The anthocyanin produced was, however, not the complete anthocyanin present in *Clitoria ternatea,* but an incomplete intermediate. This anthocyanin exhibits a violet color, but intermolecular association with flavone glucosides present in chrysanthemum resulted in blue color. Therefore, engineering of other plants with the same anthocyanin may not necessarily result in blue color if the host plant does not contain the required flavone glucosides. In addition, it would be useful to engineer the final anthocyanin present in butterfly pea rather than an incomplete intermediate, as one would expect a stronger blue color and increased stability of the anthocyanin. There is therefore a need to identify all genes of this or other blue anthocyanin biosynthetic pathways to provide the enzymes necessary to engineer complete biosynthetic pathways in heterologous hosts.

*Veronica persica (Vp)* is a native European plant of the family Plantaginaceae that produces small blue and white flowers. The anthocyanin pigment responsible for the blue color is a derivative of delphinidin-3-O-glucoside called 3-di-p-coumaroylsophoroside-5-malonylglucoside [12] or delphinidin 3-*O*-[2-*O*-(6-*O*-p-coumaroyl-glucosyl)-6-*O*-p-coumaroyl] glucoside 5-*O*-(6-*O*-malonyl)-glucoside, a compound herein referred to as "verodelphin" or "Compound VI". However, the biosynthesis of verodelphin has remained largely unknown.

Departing from the prior art, it is an object of the present invention to provide a process for the production of verodelphin and of intermediates downstream of D3G and preceding verodelphin. In particular, it is an object to provide a process for the production of Compounds III, IV, V, and VI (as defined below). It is also an object to provide genes, ORFs, and proteins involved in the biosynthetic pathway downstream of D3G to verodelphin.

### SUMMARY OF THE INVENTION

These problems are solved by:
1) A process of producing delphinidin 3-*O*-[2-*O*-(6-*O*-p-coumaroyl)-glucosyl-6-*O*-p-coumaroyl] glucoside (**IV**) from delphinidin 3-*O*-(2-*O*-glucosyl-6-*O*-p-coumaroyl) glucoside (**III**) in eukaryotic cells, comprising expressing, in said in eukaryotic cells, an acyltransferase comprising a polypeptide comprising or consisting of:
   (a) the amino acid sequence of **SEQ ID NO: 3** or
   (b) an amino acid sequence having at least 80 % sequence identity to the amino acid sequence of **SEQ ID NO: 3** or
   (c) an amino acid sequence having from 1 to 40 amino acid residue substitutions, additions, insertions and/or deletions compared to the amino acid sequence of **SEQ ID NO: 3.**
2) A process of producing delphinidin 3-*O*-[2-*O*-(6-*O*-p-coumaroyl-glucosyl)-6-O-p-coumaroyl] glucoside 5-*O*-glucoside (**V**) from delphinidin 3-O-[2-O-(6-O-p-coumaroyl)-glucosyl-6-O-p-coumaroyl] glucoside (**IV**) in eukaryotic cells, comprising expressing, in said in eukaryotic cells, a glucosyltransferase comprising a polypeptide comprising or consisting of:
   (a) the amino acid sequence of **SEQ ID NO: 4** or
   (b) an amino acid sequence having at least 80 % sequence identity to the amino acid sequence of **SEQ ID NO: 4** or
   (c) an amino acid sequence having from 1 to 40 amino acid residue substitutions, additions, insertions and/or deletions compared to the amino acid sequence of **SEQ ID NO: 4.**
3) A process of producing delphinidin 3-*O*-(2-*O*-glucosyl-6-*O*-p-coumaroyl) glucoside (**III**) from delphinidin 3-*O*-(6-*O*-p-coumaroyl)-glucoside (**II**) in eukaryotic cells, comprising expressing, in said eukaryotic cells, a glucosyltransferase comprising a polypeptide comprising or consisting of:
   (a) the amino acid sequence of **SEQ ID NO: 2** or
   (b) an amino acid sequence having at least 80 % sequence identity to the amino acid sequence of **SEQ ID NO: 2** or
   (c) an amino acid sequence having from 1 to 40 amino acid residue substitutions, additions, insertions and/or deletions compared to the amino acid sequence of **SEQ ID NO: 2.**
4) A process of producing delphinidin 3-*O*-(6-*O*-p-coumaroyl) glucoside (**II**) from delphinidin 3-O-glucoside (D3G,I) in eukaryotic cells, comprising expressing, in said eukaryotic cells, an acyltransferase comprising a polypeptide comprising or consisting of:
   (a) the amino acid sequence of **SEQ ID NO: 1** or
   (b) an amino acid sequence having at least 80 % sequence identity to the amino acid sequence of **SEQ ID NO: 1** or
   (c) an amino acid sequence having from 1 to 40 amino acid residue substitutions, additions, insertions and/or deletions compared to the amino acid sequence of **SEQ ID NO: 1.**
5) A process of producing delphinidin 3-*O*-[2-*O*-(6-*O*-p-coumaroyl-glucosyl)-6-O-p-coumaroyl] glucoside 5-*O*-(6-*O*-malonyl)-glucoside (**VI**) from delphinidin 3-*O*-[2-*O-*(6-*O*-p-coumaroyl-glucosyl)-6-*O*-p-coumaroyl] glucoside 5-*O*-glucoside (V) in eukaryotic cells, comprising expressing, in said in eukaryotic cells, a malonyltransferase comprising a polypeptide comprising or consisting of:
   (a) the amino acid sequence of **SEQ ID NO: 5** or
   (b) an amino acid sequence having at least 80 % sequence identity to the amino acid sequence of **SEQ ID NO: 5** or
   (c) an amino acid sequence having from 1 to 40 amino acid residue substitutions, additions, insertions and/or deletions compared to the amino acid sequence of **SEQ ID NO: 5.**
6) A process of producing delphinidin 3-O-[2-O-(6-O-p-coumaroyl)-glucosyl-6-O-p-coumaroyl] glucoside (**IV**) from D3G (**I**) in eukaryotic cells, comprising expressing, in said eukaryotic cells,
   (i) an acyltransferase, preferably as defined in 4) (AT1),
   (ii) a glucosyltransferase as defined in 3) (UGT94F1), and
   (iii) an acyltransferase as defined in 1) (AT2).
7) A process of producing delphinidin 3-*O*-[2-*O*-(6-*O*-p-coumaroyl-glucosyl)-6-*O*-p-coumaroyl] glucoside 5-O-glucoside (**V**) from delphinidin-3-O-glucoside (D3G; **I**) in eukaryotic cells, comprising expressing in said cells
   (i) an acyltransferase, preferably as defined in 4) (AT1),
   (ii) a glucosyltransferase as defined in 3) (UGT94F1),
   (iii) an acyltransferase as defined in 1) (AT2), and
   (iv) a glucosyltransferase as defined in 2) (5GT).
8) A process of producing the blue anthocyanin delphinidin 3-*O*-[2-*O*-(6-*O*-p-coumaroyl-glucosyl)-6-*O*-p-coumaroyl] glucoside 5-*O*-(6-*O*-malonyl)-glucoside (**VI**) from delphinidin-3-*O*-glucoside (D3G; **I**) in eukaryotic cells, comprising expressing in said cells
   (i) an acyltransferase, preferably as defined in 4),
   (ii) a glucosyltransferase as defined in 3),
   (iii) an acyltransferase as defined in 1), and
   (iv) a glucosyltransferase as defined in 2), and
   (v) a malonyltransferase, preferably as defined in 5).
9) The process according to 6) or 7), further comprising adding or providing delphinidin-3-*O*-glucoside (D3G) to said cells or providing genes to said cells allowing production of D3G in said cells.
10) A process of producing the blue anthocyanin delphinidin 3-*O*-[2-*O*-(6-*O*-p-coumaroyl-glucosyl)-6-*O*-p-coumaroyl] glucoside 5-*O*-glucoside (**V**) from delphinidin 3-*O*-(2-*O*-glucosyl-6-*O*-p-coumaroyl-glucoside) (**III**) in eukaryotic cells, comprising optionally expressing in said cells proteins that allow production of delphinidin 3-*O-*(2-*O*-glucosyl-6-*O*-p-coumaroyl-glucoside) (**III**) in said cells, and expressing in said cells
   (iii) an acyltransferase as defined in 1), and
   (iv) a glucosyltransferase as defined in 2).
11) The process according to any one of 1) to 9), wherein said cells are plant cells or yeast cells, or said plant cells are cells of a plant.
12) The process according to any one of 1) to 10), further comprising expressing a heterologous transcription factor in said cells, that promotes expression of genes preceding D3G in the biosynthetic pathway leading to D3G.
13) The process of any one of 1) to 11), wherein said expressing said gene(s) comprises providing said gene(s) to said eukaryotic cells by transformation or transfection.
14) The process of any one of 1) to 12), comprising isolating the produced product from said eukaryotic cells.
15) A protein that is an acyltransferase, comprising a polypeptide comprising or consisting of:
   (a) the amino acid sequence of **SEQ ID NO: 1** (Vp At1), or
   (b) an amino acid sequence having at least 80 % sequence identity to the amino acid sequence of **SEQ ID NO: 1** or
   (c) an amino acid sequence having from 1 to 40 amino acid residue substitutions, additions, insertions and/or deletions to the amino acid sequence of **SEQ ID NO: 1.**
16) A protein that is an acyltransferase, comprising a polypeptide comprising or consisting of:
   (a) the amino acid sequence of **SEQ ID NO: 3** (Vp At2), or
   (b) an amino acid sequence having at least 80 % sequence identity to the amino acid sequence of **SEQ ID NO: 3** or
   (c) an amino acid sequence having from 1 to 40 amino acid residue substitutions, additions, insertions and/or deletions to the amino acid sequence of **SEQ ID NO: 3.**
17) A protein that is a glycosyltransferase, comprising a polypeptide comprising or consisting of:
   (a) the amino acid sequence of **SEQ ID NO: 4** (Vp 5GT), or
   (b) an amino acid sequence having at least 80 % sequence identity to the amino acid sequence of **SEQ ID NO: 4** or
   (c) an amino acid sequence having from 1 to 40 amino acid residue substitutions, additions, insertions and/or deletions to the amino acid sequence of **SEQ ID NO: 4.**
18) A protein that is a malonyltransferase, comprising a polypeptide comprising or consisting of:
   (a) the amino acid sequence of **SEQ ID NO: 5** (Vp MAT), or
   (b) an amino acid sequence having at least 80 % sequence identity to the amino acid sequence of **SEQ ID NO: 5** or
   (c) an amino acid sequence having from 1 to 40 amino acid residue substitutions, additions, insertions and/or deletions to the amino acid sequence of **SEQ ID NO: 5.**
19) A polynucleotide encoding one or more polypeptide(s) comprising or consisting of the amino acid sequence of any one of **SEQ ID NO: 1, 3, 4,** or **5,** or
   an amino acid sequence having at least 80 % sequence identity to the amino acid sequence of any one of **SEQ ID NO: 1, 3, 4,** or **5,** or
   an amino acid sequence having from 1 to 40 amino acid residue substitutions, additions, insertions and/or deletions to the amino acid sequence of any one of **SEQ ID NO: 1, 3, 4,** or **5;** or
   a polynucleotide comprising or consisting of the nucleotide sequence of **SEQ ID NO: 6, 8, 9,** and/or **10.**
20) The polynucleotide according to 19), comprising a coding sequence encoding a polypeptide(s) comprising or consisting of the amino acid sequence selected from any one of **SEQ ID NO: 1, 3, 4,** or **5** and, linked by a coding sequence encoding a 2A peptide, a further coding sequence encoding another polypeptide(s) comprising or consisting of the amino acid sequence selected from any one of **SEQ ID NO: 1, 3, 4,** or **5.**
21) A vector or recombinant construct comprising the polynucleotide of 19) or 20).
22) A plant or plant cell transfected or transformed with a polynucleotide according to 19) or 20), or with a vector or construct according to 21).
23) The plant or plant cell according to 22), which is other than a *Veronica persica* plant or plant cell, such as tomato.
24) The plant or plant cell according to 22) or 23), comprising a gene encoding the protein as defined in 16) (AT2) and a gene encoding the protein as defined in 17) (5GT).
25) The plant or plant cell according to any one of 22) to 24), further comprising one or more genes allowing production of D3G in said plant or plant cell, such as Rosea1 and/or Delila.
26) The plant or plant cell according to any one of 22) to 25), having a gene encoding rhamnosyltransferase activity silenced or inactivated.

The inventors have found four genes, and the encoded proteins, involved in biosynthesis of the blue anthocyanin of *Veronica persica* downstream of D3G. These genes and proteins are two acyltransferases, Vp AT1 and Vp AT2, a glucosyltransferase, Vp 5GT, and a malonyltransferase, Vp MAT. Expression of these four genes with a 5th gene previously identified by another group (UGT94F1) enables the biosynthesis of the blue anthocyanin verodelphin (herein: Compound **VI** of formula **VI** or a neutral or anionic form thereof) in heterologous hosts. This process, and the genes as well as proteins encoded by the genes, can be used for engineering eukaryotic cells or plants with blue flowers or blue fruits, such as tomato. The blue fruits can serve as a source material for production of natural blue food colorant. The genes can also be used to engineer microorganisms such as yeast for production of blue food colorant.

### BRIEF DESCRIPTION OF THE DRAWINGS

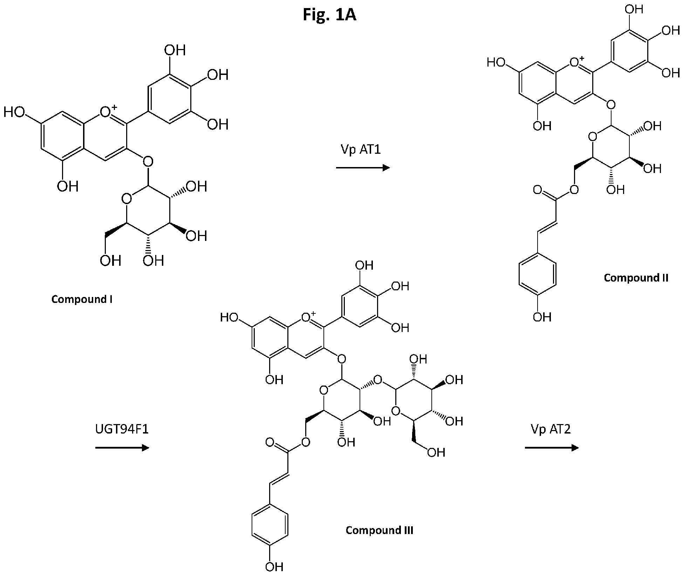

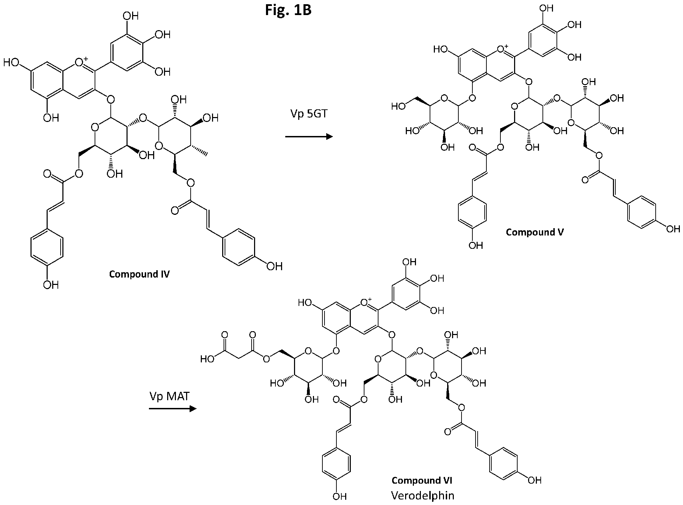

**Fig. 1A****, B**: Structure of verodelphin (Compound **VI**) and its precursors starting from delphinidin-3-O-glucoside (D3G, Compound **I**). Fives genes are required for biosynthesis of verodelphin from delphinidin-3-O-glucoside in *Veronica persica.* Vp AT1, Vp UGT94F1, Vp AT2, Vp 5GT, Vp MAT. The order with which the enzymes contribute to verodelphin biosynthesis is as indicated. Vp stands for *Veronica persica.* AT stands for acyltransferase. 5GT stands for glucosyltransferase. MAT stands for malonyltransferase. Compounds **I** to **VI** are shown in their protonated, cationic forms. Counter-anions are not shown, as they are not particularly limited.

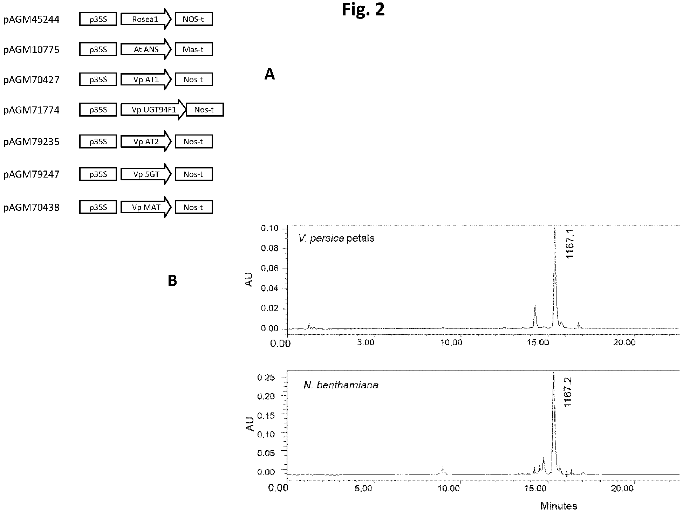

**Fig. 2****:** Biosynthesis of verodelphin by transient expression in *Nicotiana benthamiana.* **(A)** Structure of expression constructs used for verodelphin biosynthesis in *N. benthamiana* line 29-2. (**B**) All constructs were delivered by *Agrobacterium-mediated* infiltration to leaves of *N. benthamiana* line 29-2. Five days after infiltration, an extract from the infiltrated leaf area was made. LC-MS analysis revealed the presence of a peak with a [M + H]⁺ ion at m/z 1167 characteristic for verodelphin. The same peak is observed from an extract made from *Veronica persica* petals.

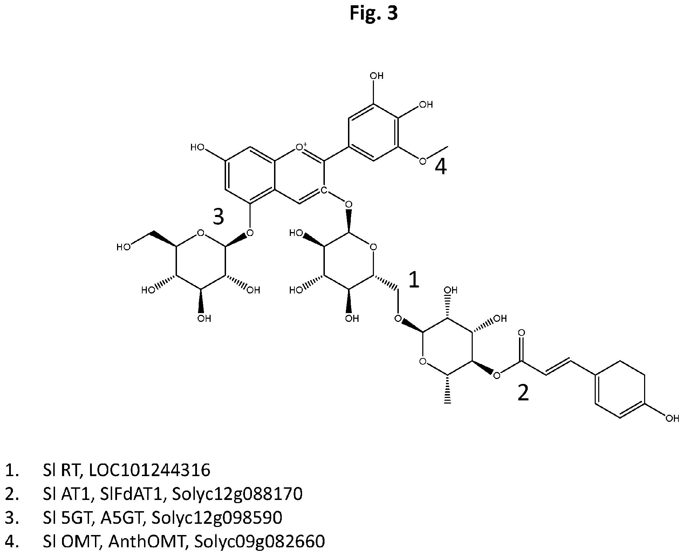

**Fig. 3****:** Structure of native tomato anthocyanin petunidin-3-O-[4-(trans-p-coumaroyl)-rutinoside]-5-O-β-D-glucoside. Four genes are required for biosynthesis of the native tomato anthocyanin from delphinidin-3-*O*-glucoside: SI RT, SI AT1, SI 5GT, and SI OMT. Numerals in the structure indicate the positions where the moieties are introduced by the respective gene products given.

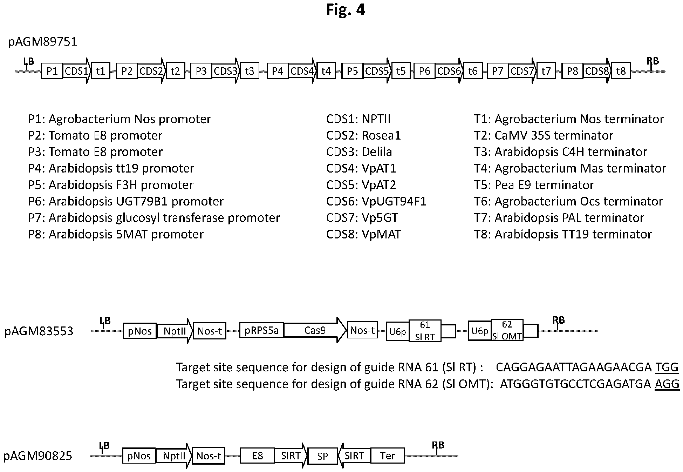

**Fig. 4****:** Structure of constructs used for verodelphin biosynthesis in tomato. LB stands for T-DNA left border. RB stands for T-DNA right border. P stands for promoter. CDS stands for coding sequence. T stands for transcription terminator. Target site sequence for guide RNA 61 is **SEQ ID NO: 12.** Target site sequence for guide RNA 62 is **SEQ ID NO: 13.** Underlined triplets indicate the PAM (protospacer adjacent motif) at the target site.

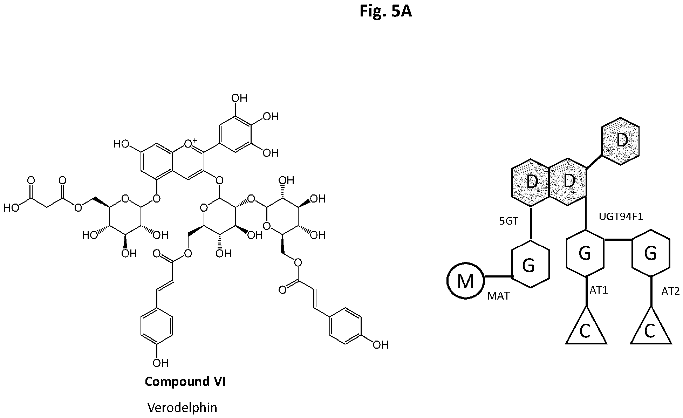

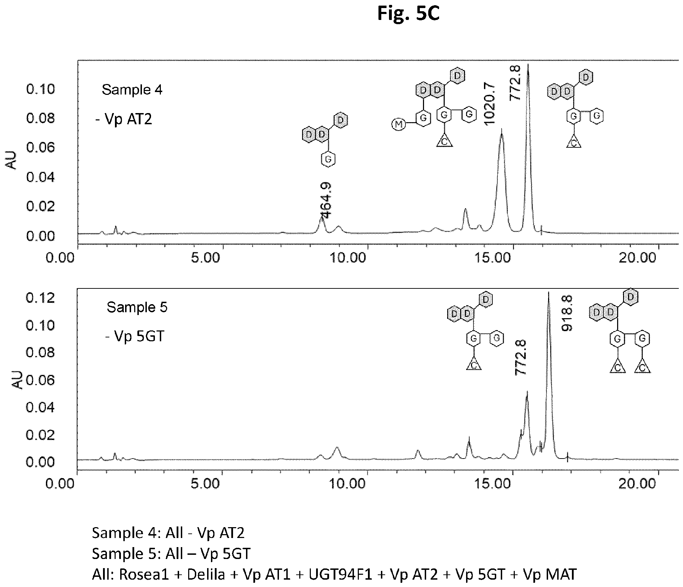

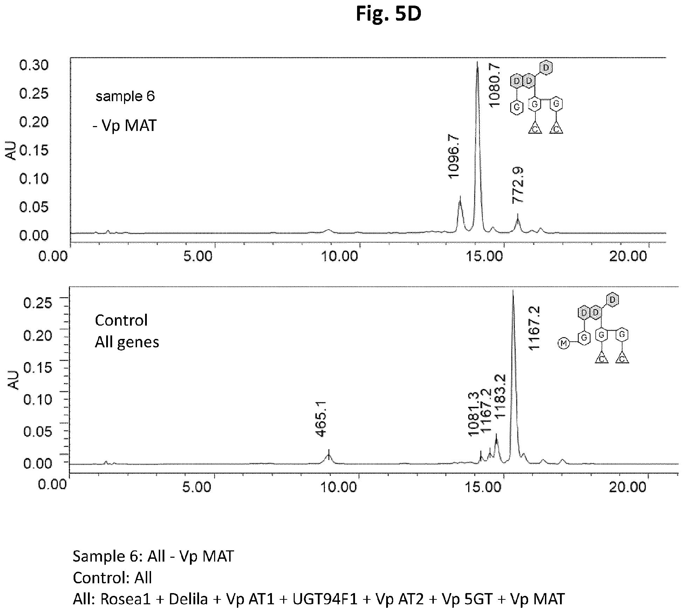

**Fig. 5 A-D:** Determination of the gene order in the production of verodelphin in *Veronica persica.* (**A**): Explanation of the experimental strategy. Structure of verodelphin (left) and schematic representation of verodelphin (right). The 3 hexagons marked with D represent the delphinidin core chromophore. G, C and M represent glucose, coumaroyl and malonyl residues, respectively. In transient transfection (infiltration) experiments, either all five genes are provided to plant leaves or selected genes are left out to determine the products formed by LC-MS. (**B**) Results of LC-MS analysis of infiltrated leaf areas where the genes left out were: Vp AT1 (top) and UGT94F1 (bottom). (**C**) Results of LC-MS analysis of infiltrated leaf areas where the genes left out were: VP AT2 (top) and VP 5GT (bottom). (**D**) Results of LC-MS analysis of infiltrated leaf areas where the genes left out were: Vp MAT (top). Bottom: all genes indicated at the bottom were transfected into leaves. Results: Leaving out AT1 results in remaining D3G with little formation of downstream delphinidin derivatives. Leaving out UGT94F2 leads to formation of Compound II. Leaving out AT2: no Compound **IV** is formed. Leaving out 5GT: no Compound **V** is formed. (F) Leaving out MAT: no Compound **VI** is formed.

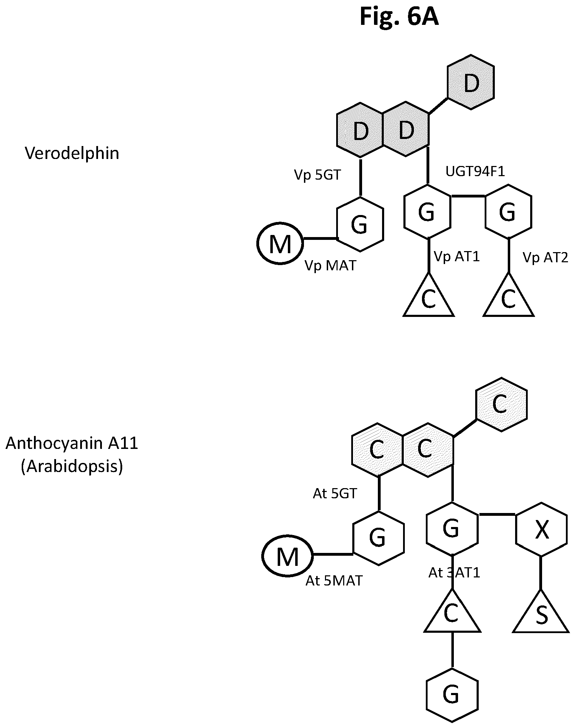

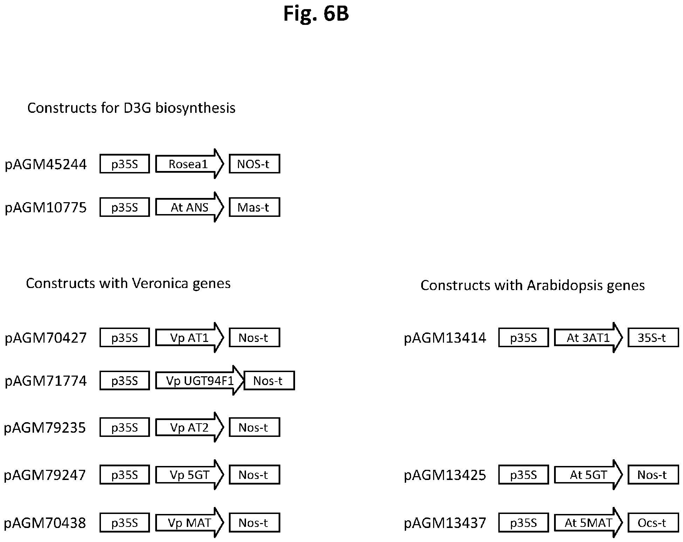

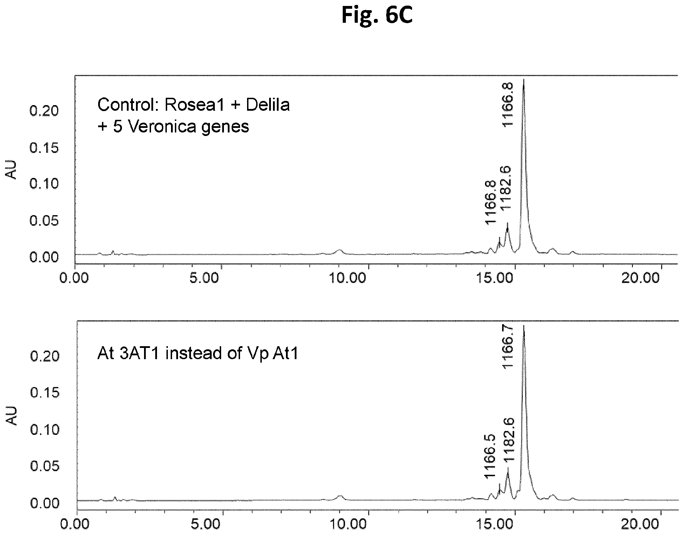

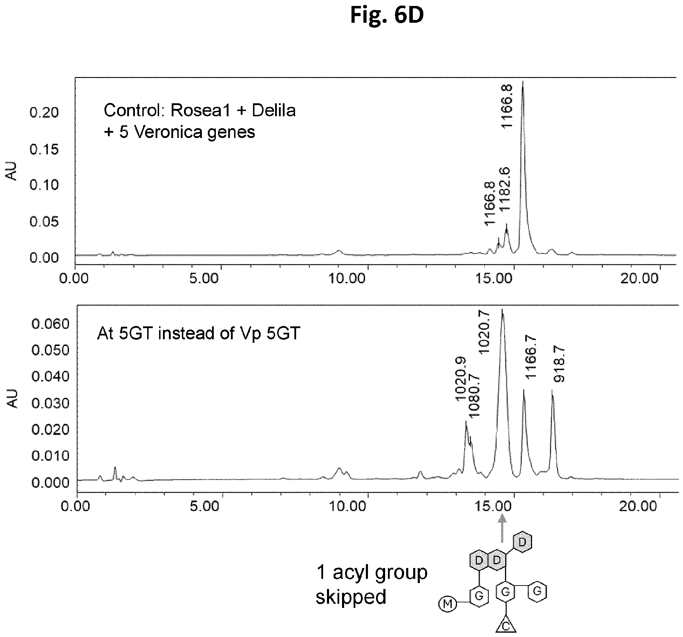

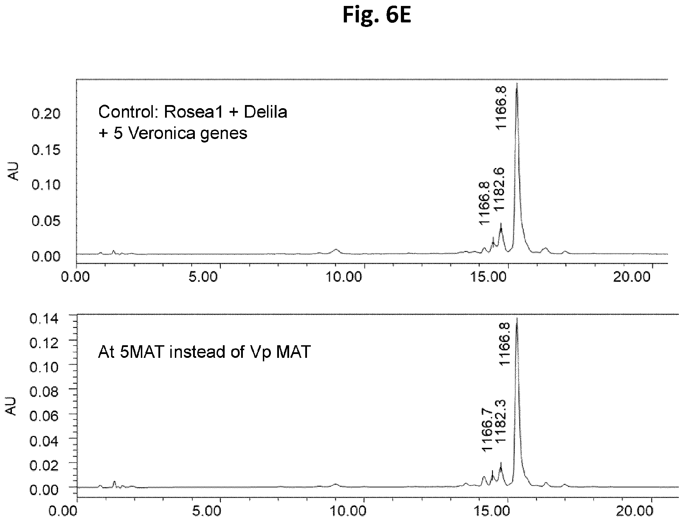

**Fig. 6** Investigation whether some of the enzymes of the biosynthetic pathway between D3G and verodelphin can be substituted by *Arabidopsis* enzymes. (**A**) Schematic structure of verodelphin (top). The 3 hexagons marked with D represent the delphinidin core chromophore. G, C and M represent glucose, coumaroyl and malonyl residues, respectively. Bottom: Schematic structure of anthocyanin A11 (*Arabidopsis*; structure explained in Fig. 6(c) of Ref. 15). The 3 hexagons marked with C represent a cyanidin core chromophore. G, X, C, M and S represent glucose, xylose, coumaroyl, malonyl, and synapoyl residues, respectively. (**B**) Outline of constructs used for infiltration of *N*. *benthamiana* leaves. (**C**) LC-MS analysis of extracts from infiltrated leaf areas. The gene infiltrated are indicated. Replacement of *Vp* AT1 by *Arabidopsis* AT1: production of verodelphin was maintained. (**D**) LC-MS analysis of extracts from infiltrated leaf areas. The gene infiltrated are indicated. Replacement of *Vp* 5GT by *Arabidopsis* 5GT: production of verodelphin was not maintained. (**E**) LC-MS analysis of extracts from infiltrated leaf areas. The gene infiltrated are indicated. Replacement of *Vp* MAT by *Arabidopsis* MAT: production of verodelphin was maintained.

### DETAILED DESCRIPTION OF THE INVENTION

*Veronica persica (Vp)* is a native European plant of the family *Plantaginaceae* that produces small blue and white flowers. The anthocyanin pigment responsible for the blue color is a derivative of delphinidin-3-*O*-glucoside called 3-di-p-coumaroylsophoroside-5-malonylglucoside [12] or delphinidin 3-*O*-[2-*O*-(6-*O*-p-coumaroyl-glucosyl)-6-O-p-coumaroyl] glucoside 5-*O*-(6-*O*-malonyl)-glucoside that is herein referred to as "verodelphin" or Compound **VI** for brevity (**Fig. 1**).

Biosynthesis of this compound, starting from delphinidin-3-O-glucoside (D3G; Compound I), requires 5 enzymatic steps, as shown in **Fig. 1****.** One of the enzymes involved in biosynthesis of verodelphin was previously identified [13]. This enzyme, UGT94F1 (GenBank AB514127), was reported to add a glucosyl residue at the 2" position of the glucosyl residue of D3G [13]. The inventors of the present invention have surprisingly found that, in fact, this enzyme does not use D3G as a substrate but instead uses delphinidin 3-O-(6-O-p-coumaroyl) glucoside (Compound II) as substrate. Therefore, the enzymatic reaction catalyzed by UGT94F1 represents, departing from D3G, the 2^{nd} enzymatic reaction step of this pathway leading to verodelphin and not the first one **(****Fig. 1****).**

The inventors have made a transcriptome from RNAs extracted from *Veronica persica* leaves and petals. The transcriptome was screened for the presence of genes that might be involved in anthocyanin biosynthesis (glucosyltransferases and acyltransferases, including coumaroyltransferases and malonyltransferases), and that were expressed at high level in petals and lower level in leaves. cDNAs of candidate genes were amplified by PCR and subcloned in an expression vector under control of the 35S promoter (cloning vector pAGM53151, Addgene Plasmid #169093) using TEDA cloning [14]. Coding sequences of the genes were amplified from cDNAs by PCR using primers containing a 5' extension homologous to the vector pAGM53151. Sequence of the primer pairs used for amplification of the 5 genes Vp AT1, UGT94F1, Vp AT2, Vp 5Gt and Vp MAT are:
VpAT1: *catttacaattatcgatac* aatgccaacaacacctacaataagtgtc (**SEQ ID NO: 16**) / *gcttgactctagaggatc* aagcttatagtttcagaccctccacgaag (**SEQ ID NO: 17**),
UGT94F1: *catttacaattatcgatac* aatggagaaagaagaagcaaaaatgttcaaaattc (**SEQ ID NO: 18**) / *gcttgactctagaggatc* aagctcagtcacattgttgcaacttgttcttcg (**SEQ ID NO: 19**),
Vp AT2: *catttacaattatcgatac* aatggctactacacccgcaaagcg (**SEQ ID NO: 20**) / *gcttgactctagaggatc* aagctagtttagtccttcggagaagatagacgc (**SEQ ID NO: 21**),
Vp 5GT: *catttacaattatcgatac* aatggcgggacctcgaatcttagtagc (**SEQ ID NO: 22**) / *gcttgactctagaggatc* aagcctactgagaaactgcaatgacttcatc (**SEQ ID NO: 23**),
Vp MAT: *catttacaattatcgatac* aatgactatctccacaaacaaagtaactgtcc (**SEQ ID NO: 24**) / *gcttgactctagaggatc* aagcttacttctccaacccattagcgaaaacag (**SEQ ID NO: 25**),
with all sequences shown in italics being homologous to vector sequences. The PCR products were purified with a column and cloned in vector pAGM53151 digested with Bsal using the TEDA enzyme mix. All clones were sequenced. Correct constructs with either the same sequence as identified from the transcriptome, or with some sequence polymorphisms found in several sequenced clones were selected. These clones were transformed in *Agrobacterium* strain GV3101:pMP90. The genes were then transiently expressed in *Nicotiana benthamiana* leaves together with two genes previously shown to be sufficient for inducing biosynthesis of D3G in Nicotiana benthamiana leaves [15]. These 2 genes consist of the MYB transcription factor Rosea1 from *Antirrhinum majus* and an *Arabidopsis thaliana* anthocyanidin synthase (At ANS). Transient expression of these two genes in wildtype *Nicotiana benthamiana* results in biosynthesis of delphinidin-3-O-rutinoside, which is not the precursor of verodelphin biosynthesis. In contrast, expression of these two genes in a *N. benthamiana* mutant line that lacks anthocyanin-3-O-glucoside rhamnosyltransferase activity (line Nb 29-2) leads to biosynthesis of D3G [15], which is the substrate needed. Candidate genes from *Veronica* were therefore transiently co-expressed with Rosea1 and At ANS in *Nicotiana benthamiana* line Nb 29-2.

The inventors have surprisingly identified the genes coding for all four previously unidentified enzymes of the pathway. These are two acyltransferases, Vp AT1 (SEQ ID NOs: 1, 6) and Vp AT2 (SEQ ID NOs: 3, 8), a glucosyltransferase, Vp 5GT (SEQ ID NOs: 4, 9), and a malonyltransferase, Vp MAT (SEQ ID NOs: 5, 10). The inventors have found that the five enzymatic steps take place in the following order with enzymes Vp AT1, UGT94F1, VpAT2, Vp 5GT, and finally Vp MAT, as described in **Fig. 1****.** Some of these enzymes can be replaced by homologous enzymes from plants other than *Veronica.*

The genes for UGT94F1, Vp 5GT and Vp MAT do not contain any introns, while the genes for VpAT1 and VpAT2 each contain one intron. Coexpression of the five genes in organisms producing D3G leads to biosynthesis of verodelphin. Therefore, to produce verodelphin in heterologous hosts, such as eukaryotic cells, the target organism or cells may, if necessary, first be engineered to be able to produce D3G and then further be engineered to express the five *Veronica persica* genes or suitable variants thereof in the desired organ or tissue. The invention also provides processes, and proteins, genes and ORFs therefor, for the production of intermediates in the biosynthetic pathway to verodelphin downstream of D3G, such as Compound IV and Compound V.

Each of Compounds **I** to **VI** shown in **Fig. 1** comprises the cation shown and a counter-anion that is not shown in **Fig. 1****.** The cations shown in **Fig. 1** each has a delphinidin moiety comprising a flavylium moiety and are therefore cations. Cationic delphinidin comprising a flavylium moiety has the following structure:

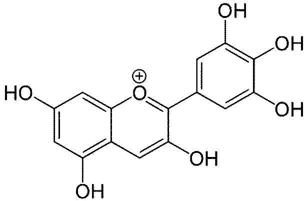

The counter-anions of Compounds **I** to **VI** shown in **Fig. 1** are not specifically limited and may be any conjugated base of an inorganic or organic acid, such as chloride, phosphate, formate, acetate or any other carboxylate. The skilled person understands that the protonation state (and thus charge) of the delphinidin moieties of Compounds **I** to **VI** depends in aqueous solution on the pH. In the present invention, Compounds I to **VI** are produced in eukaryotic cells. Thus, the pH and the charge state of the delphinidin moieties depend on the conditions in the cells or tissue in which they are produced. Accordingly, the invention is not limited with regard to pH and also not with regard to the protonation/deprotonation state of the delphinidin moieties of Compounds I to **VI.** Instead of Compounds **I** to **VI** with cationic delphinidin moieties shown in **Fig. 1****,** the compounds having deprotonated charge-neutral delphinidin moieties or anionic delphinidin moieties may be employed in the invention. Compounds **I** to **VI** with anionic delphinidin moieties may have a cationic counter-ion that is not particularly limited, such as an alkaline metal ion, e.g. a sodium or potassium ion, or ammonium. Herein, the term "Compound **VI"** is generic and covers the compounds having cationic, charge-neutral and anionic delphinidin moieties. This applies analogously to Compounds I to **V.**

Where, herein, reference is made to the cation of (any one of) formula (**I**) to (**VI**), the specific cation shown in **Fig. 1** for the respective cation is meant. The wording that a compound indicated by a specific name "comprises the cation of formula ..." means that the compound generally also comprises an anion that is not identified.

The chemical names of Compounds **I** to **VI** are, as generally used in the literature, as follows. As explained above, the protonation state of the delphinidin moieties are not specified in these names and are, insofar, generic.
Compound **I**: delphinin 3-*O*-glucoside; Exact mass: 465.10, Molecular weight: 465.39
Compound **II**: delphinidin 3-O-(6-O-p-coumaroyl) glucoside; Exact mass: 611.14, Molecular weight: 611.53
Compound **III**: delphinidin 3-*O*-(2-*O*-glucosyl-6-*O*-p-coumaroyl) glucoside; Exact mass: 773.19, Molecular weight: 773.67
Compound **IV:** delphinidin 3-*O*-[2-*O*-(6-*O*-p-coumaroyl)-glucosyl-6-*O*-p-coumaroyl] glucoside; Exact mass: 917.25, Molecular weight: 917.85
Compound **V:** delphinidin 3-*O*-[2-*O*-(6-*O*-p-coumaroyl-glucosyl)-6-*O*-p-coumaroyl] glucoside 5-*O*-glucoside; Exact mass: 1081.28
Compound **VI**: delphinidin 3-*O*-[2-*O*-(6-*O*-p-coumaroyl-glucosyl)-6-*O*-p-coumaroyl] glucoside 5-O-(6-O-malonyl)-glucoside; Exact mass: 1167.28.

These structures are generally indicated in the cationic form in the literature.

The blue color of verodelphin intended in the present invention is seen in aqueous solution in the vicinity of pH 7, but the pH of different cell organelles can vary between organelles and between different cells. The colors, structures, and pH dependencies of various anthocyanins are discussed in the review of Houghton et al., Plants 2021, 10, 726; doi.org/10.3390/plants10040726.

The five reaction steps shown in **Fig. 1** may be enzymatically catalyzed by the proteins (enzymes) capable of performing these reactions in the presence of the substrates involved. These proteins may be the proteins comprising a polypeptide comprising or consisting of the amino acid sequence of SEQ ID NOs: 1 to 5 or the variants thereof defined herein. These five proteins and their variants are referred to herein as "proteins of the invention" or "enzymes of the invention". The proteins comprising a polypeptide comprising or consisting of the amino acid sequence of SEQ ID NOs: 1 to 5 (i.e. excluding variants as defined below) are referred to herein as "parent proteins". The wording "protein comprising a polypeptide" means that the protein may include, apart from the polypeptide, metal ions or other co-factors, disulfide bridges, post-translational modifications, or may be multimers of the polypeptide in terms of the quaternary structure of the protein. For the proteins of the invention, the term "enzymes" is used interchangeably, since these proteins are enzymes. Thus, the respective function of each of these enzymes of catalyzing the reactions explained herein (**Fig. 1** and below) is implicit in the term "proteins of the invention".

Instead of the protein (or enzyme) comprising a polypeptide comprising or consisting of the amino acid sequence of **SEQ ID NO: 1** to **5** ("parent protein" or "parent enzyme"), an enzyme comprising a polypeptide comprising or consisting of an amino acid sequence having at least 80 %, preferably at least 85 %, more preferably at least 90 %, and even more preferably at least 95 % sequence identity to the amino acid sequence of **SEQ ID NO: 1** to **5,** respectively, may be used, whereby these variants have the enzymatic activity for catalyzing the respective reaction of the respective parent enzyme.

Instead of the enzyme comprising a polypeptide comprising or consisting of the amino acid sequence of **SEQ ID NO: 1** to **5** ("parent enzyme"), an enzyme comprising a polypeptide comprising or consisting of an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 1** to **5,** respectively, may be used, whereby these variants have the enzymatic activity for catalyzing the respective reaction of the respective parent enzyme. These proteins will be described in further detail in the following.

Herein, the determination of amino acid sequence identities is done using Align Sequences Protein BLAST (BLASTP 2.6.1+) (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.).

Where a protein is defined herein by a number or number range of amino acid residue substitutions, additions, insertions and/or deletions, amino acid residue substitutions, additions, insertions and deletions may be combined, but the given number or number range refers to the sum of all amino acid residue substitutions, additions, insertions and deletions. Among amino acid residue substitutions, additions, insertions and deletions, amino acid substitutions, additions, and deletions are preferred. The term "insertion" relates to insertions within the amino acid sequence of a reference sequence, i.e. excluding additions at the C- or N-terminal end. The term additions means additions at the C- or N-terminal end of the amino acid sequence of a reference sequence. A deletion may be a deletion of a terminal or an internal amino acid residue of a reference sequence.

The protein of **SEQ ID NO: 1** (also referred to herein as Vp At1) is an acyltransferase capable of transferring a p-coumaryol group from p-hydroxycinnamoyl-CoA to D3G (Compound I), a compound comprising the cation of formula **I** or a neutral or anionic form thereof, to form Compound **II,** a compound comprising the cation of formula **II** or a neutral or anionic form thereof. The co-substrate p-hydroxycinnamoyl-CoA is generally naturally present in eukaryotic cells, such as plant or yeast cells, whereby additional genetic engineering for providing the co-substrate is possible, but normally not needed. Apart from
(a) the acyltransferase comprising a polypeptide comprising or consisting of the amino acid sequence of **SEQ ID NO: 1,** the invention also provides acyltransferase variants comprising a polypeptide comprising or consisting of
(b) an amino acid sequence having at least 80 % sequence identity to the amino acid sequence of **SEQ ID NO: 1** (with the preferred minimum sequence identity values given above) or
(c) an amino acid sequence having from 1 to 40 amino acid residue substitutions, additions, insertions and/or deletions to the amino acid sequence of **SEQ ID NO: 1** (with the preferred values given above). These variants are capable of catalyzing the reaction of Compound **I** (a compound comprising the cation of formula **I** or a neutral or anionic form thereof) with p-hydroxycinnamoyl-CoA to Compound **II** (a compound comprising the cation of formula **II** or a neutral or anionic form thereof).

Accordingly, the invention provides a process of producing delphinidin 3-O-(6-O-p-coumaroyl) glucoside comprising the cation of formula (**II**) or a neutral form or anionic form thereof from delphinidin 3-O-glucoside (D3G; a compound comprising the cation of formula I or a neutral form or anionic form thereof) in eukaryotic cells, comprising expressing, in said eukaryotic cells, an acyltransferase, or an ORF or gene encoding such acyl transferase, comprising a polypeptide comprising or consisting of:
(a) the amino acid sequence of **SEQ ID NO: 1** or
(b) an amino acid sequence having at least 80 %, preferably at least 85 %, more preferably at least 90 %, and even more preferably at least 95 % sequence identity to the amino acid sequence of **SEQ ID NO: 1** or
(c) an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 1.**

Herein, the wording expressing an ORF (open reading frame) or gene means that the encoded protein is expressed in the eukaryotic cells.

The protein of **SEQ ID NO: 2** (UGT94F1) is a glycosyltransferase (more specifically: UDP-glucose-dependent glucosyltransferase) capable of transferring a glucose group from UDP-glucose to Compound **II,** a compound comprising the cation of formula **II** or a neutral or anionic form thereof, to form Compound **III**, a compound comprising the cation of formula **III** or a neutral or anionic form thereof. The co-substrate UDP-glucose is generally naturally present in eukaryotic cells, such as plant or yeast cells, whereby an additional genetic engineering for providing the co-substrate is possible, but normally not needed. Contrary to Ono *et al.* [13], the inventors have found that this enzyme uses Compound **II** as substrate. Apart from (a) the glycosyltransferase comprising a polypeptide comprising or consisting of the amino acid sequence of **SEQ ID NO: 2,** the invention also provides glycosyltransferase variants comprising a polypeptide comprising or consisting of
(b) an amino acid sequence having at least 80 % sequence identity to the amino acid sequence of **SEQ ID NO: 2** (with the preferred minimum sequence identity values given above) or
(c) an amino acid sequence having from 1 to 40 amino acid residue substitutions, additions, insertions and/or deletions to the amino acid sequence of **SEQ ID NO: 2** (with the preferred values given above).
These variants are capable of catalyzing the reaction of Compound **II** (a compound comprising the cation of formula **II** or a neutral or anionic form thereof) with UDP-glucose to Compound **III** (a compound comprising the cation of formula **III** or a neutral form or anionic form thereof).

Accordingly, the invention provides a process of producing delphinidin 3-*O*-(2-*O-*glucosyl-6-*O*-p-coumaroyl) glucoside comprising the cation of formula (**III**) or a neutral form or anionic form thereof from delphinidin 3-O-(6-O-p-coumaroyl) glucoside comprising the cation of formula (**II**) or a neutral form or anionic form thereof in eukaryotic cells, comprising expressing, in said eukaryotic cells, a glycosyltransferase, or an ORF or gene encoding such glycosyltransferase, comprising a polypeptide comprising or consisting of:
(a) the amino acid sequence of **SEQ ID NO: 2** or
(b) an amino acid sequence having at least 80 %, preferably at least 85 %, more preferably at least 90 %, and even more preferably at least 95 % sequence identity to the amino acid sequence of **SEQ ID NO: 2** or
(c) an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 2.**

The protein of **SEQ ID NO: 3** (Vp AT2) is an acyltransferase capable of transferring an p-coumaryol group from p-hydroxycinnamoyl-CoA to Compound **III**, a compound comprising the cation of formula **III** or a neutral or anionic form thereof, to form Compound **IV,** a compound comprising the cation of formula **IV** or a neutral or anionic form thereof. The co-substrate p-hydroxycinnamoyl-CoA is generally naturally present in eukaryotic cells, such as plant or yeast cells, whereby an additional genetic engineering for providing the co-substrate is possible, but normally not needed. Apart from (a) the acyltransferase comprising a polypeptide comprising or consisting of the amino acid sequence of **SEQ ID NO: 3,** the invention also provides acyltransferase variants comprising a polypeptide comprising or consisting of
(b) an amino acid sequence having at least 80 % sequence identity to the amino acid sequence of **SEQ ID NO: 3** (with the preferred minimum sequence identity values given above) or
(c) an amino acid sequence having from 1 to 40 amino acid residue substitutions, additions, insertions and/or deletions to the amino acid sequence of **SEQ ID NO: 3** (with the preferred values given above). These variants are capable of catalyzing the reaction of Compound **III** with p-hydroxycinnamoyl-CoA to Compound **IV.**

Accordingly, the invention provides a process of producing delphinidin 3-*O*-[2-*O*-(6-*O*-p-coumaroyl)-glucosyl-6-*O*-p-coumaroyl] glucoside comprising the cation of formula **IV** or a neutral form or anionic form thereof from delphinidin 3-*O*-(2-*O*-glucosyl-6-*O*-p-coumaroyl) glucoside comprising the cation of formula (**III**) or a neutral form or anionic form thereof in eukaryotic cells, comprising expressing, in said eukaryotic cells, an acyltransferase, or an ORF or gene encoding such acyltransferase, comprising a polypeptide comprising or consisting of:
(a) the amino acid sequence of **SEQ ID NO: 3** or
(b) an amino acid sequence having at least 80 %, preferably at least 85 %, more preferably at least 90 %, and even more preferably at least 95 % sequence identity to the amino acid sequence of **SEQ ID NO: 3** or
(c) an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 3.**

In said process, Compound **IV** is generally produced in said eukaryotic cells, preferably in plant cells or cells of a plant.

The protein of **SEQ ID NO: 4** (Vp 5GT) is an glycosyltransferase (more specifically: UDP-glucose-dependent glucosyltransferase) capable of transferring a glucose group from UDP-glucose to the 5-hydroxy group of the benzopyran moiety of Compound IV, a compound comprising the cation of formula **IV** or a neutral or anionic form thereof, to form Compound **V,** a compound comprising the cation of formula **V** or a neutral or anionic form thereof. The co-substrate UDP-glucose is generally naturally present in eukaryotic cells, such as plant or yeast cells, whereby an additional genetic engineering for providing the co-substrate is possible, but normally not needed. Apart from (a) the glycosyltransferase comprising a polypeptide comprising or consisting of the amino acid sequence of **SEQ ID NO: 4,** the invention also provides glycosyltransferase variants comprising a polypeptide comprising or consisting of
(b) an amino acid sequence having at least 80 % sequence identity to the amino acid sequence of **SEQ ID NO: 4** (with the preferred minimum sequence identity values given above) or
(c) an amino acid sequence having from 1 to 40 amino acid residue substitutions, additions, insertions and/or deletions to the amino acid sequence of **SEQ ID NO: 4** (with the preferred values given above). These variants are capable of catalyzing the reaction of Compound IV with UDP-glucose to Compound **V.**

Accordingly, the invention provides a process of producing delphinidin 3-*O*-[2-*O*-(6-*O*-p-coumaroyl-glucosyl)-6-*O*-p-coumaroyl] glucoside 5-O-glucoside comprising the cation of formula (V) or a neutral form or anionic form thereof from delphinidin 3-*O*-[2-*O*-(6-*O*-p-coumaroyl)-glucosyl-6-*O*-p-coumaroyl] glucoside comprising the cation of formula (IV) or a neutral form or anionic form thereof in eukaryotic cells, comprising expressing, in said eukaryotic cells, a glycosyltransferase, or an ORF or gene encoding such glycosyltransferase, comprising a polypeptide comprising or consisting of:
(a) the amino acid sequence of **SEQ ID NO: 4** or
(b) an amino acid sequence having at least 80 %, preferably at least 85 %, more preferably at least 90 %, and even more preferably at least 95 % sequence identity to the amino acid sequence of **SEQ ID NO: 4** or
(c) an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 4.**

The protein of **SEQ ID NO: 5** (Vp MAT) is a malonyltransferase capable of transferring a malonyl group from malonyl-CoA to the cation of formula **V** or a neutral or anionic form thereof, notably to the 6-hydroxy group of the glucose moiety at the 5-position of the benzopyran moiety of Compound **V,** a compound comprising the cation of formula **V** or a neutral or anionic form thereof. The co-substrate malonyl-CoA is an intermediate of the fatty acid metabolism and is therefore generally naturally present in eukaryotic cells, such as plant or yeast cells, whereby an additional genetic engineering for providing the co-substrate is possible, but normally not needed. Apart from (a) the malonyltransferase comprising a polypeptide comprising or consisting of the amino acid sequence of **SEQ ID NO: 5,** the invention also provides malonyltransferase variants comprising a polypeptide comprising or consisting of
(b) an amino acid sequence having at least 80 % sequence identity to the amino acid sequence of **SEQ ID NO: 5** (with the preferred minimum sequence identity values given above) or
(c) an amino acid sequence having from 1 to 40 amino acid residue substitutions, additions, insertions and/or deletions to the amino acid sequence of SEQ ID **NO: 5** (with the preferred values given above).
These variants are capable of catalyzing the reaction of Compound **V** with malonyl-CoA to Compound **VI**.

Accordingly, the invention provides a process of producing delphinidin 3-*O*-[2-*O*-(6-O-p-coumaroyl-glucosyl)-6-*O*-p-coumaroyl] glucoside 5-*O*-(6-*O*-malonyl)-glucoside comprising the cation of formula (VI) or a neutral form or anionic form thereof from delphinidin 3-*O*-[2-*O*-(6-*O*-p-coumaroyl-glucosyl)-6-*O*-p-coumaroyl] glucoside 5-*O*-glucoside comprising the cation of formula **(V)** or a neutral form or anionic form thereof in eukaryotic cells, comprising expressing, in said eukaryotic cells, a malonyltransferase, or an ORF or gene encoding such malonyltransferase, comprising a polypeptide comprising or consisting of:
(a) the amino acid sequence of **SEQ ID NO: 5** or
(b) an amino acid sequence having at least 80 %, preferably at least 85 %, more preferably at least 90 %, and even more preferably at least 95 % sequence identity to the amino acid sequence of **SEQ ID NO: 5** or
(c) an amino acid sequence having from 1 to 40, preferably from 1 to 30, more preferably from 1 to 20, and even more preferably from 1 to 10 amino acid residue substitutions, additions, insertions and/or deletions compared to the amino acid sequence of **SEQ ID NO: 5.**

Depending on the eukaryotic cells used, these may need to be genetically-modified to be able to produce the reactants of the above reactions, i.e. the substrates of the respective enzymes. Accordingly, eukaryotic cells may have to express the ORFs or genes (and thus produce the protein) required for producing the respective reactants, notably Compounds **II** to **V** or neutral or anionic forms thereof.

Accordingly, the invention also provides a process of producing the blue anthocyanin verodelphin (Compound **VI**) from Compound **I** (D3G) in eukaryotic cells, comprising expressing in said cells
(i) an acyltransferase, or an ORF or gene encoding it, capable of transferring a p-coumaryol group from p-hydroxycinnamoyl-CoA to D3G to produce Compound II, such as the acyltransferase defined above with reference to **SEQ ID NO: 1,**
(ii) a glucosyltransferase, or an ORF or gene encoding it, as defined above with reference to **SEQ ID NO: 2,**
(iii) an acyltransferase, or an ORF or gene encoding it, as defined above with reference to **SEQ ID NO: 3,** and
(iv) a glucosyltransferase, or an ORF or gene encoding it, as defined above with reference to **SEQ ID NO: 4,** and
(v) a malonyltransferase, or an ORF or gene encoding it, capable of transferring a malonyl group from malonyl-CoA to Compound **V** to produce Compound **VI**, such as the malonyltransferase defined above with reference to **SEQ ID NO: 5.**

The wording "defined with reference to SEQ ID NO: x" means that the enzyme comprises a polypeptide comprising or consisting of the amino acid sequence of SEQ ID NO: x or the variants defined in items (b) and (c) above.

The acyltransferase, or an ORF or gene encoding it, capable of transferring a p-coumaryol group from p-hydroxycinnamoyl-CoA to D3G to produce Compound **II** may be that defined above with reference to **SEQ ID NO: 1** or another acyltransferase capable of catalyzing this reaction, such as At1 from *Arabidopsis* described in the Examples. The Arabidopsis locus in TAIR is AT1G03940 and the genomic sequence in Genbank is locus NM_100275.

The malonyltransferase, or an ORF or gene encoding it, capable of transferring a malonyl group from malonyl-CoA to Compound **V** to produce Compound **VI** may be that defined above with reference to **SEQ ID NO: 5** or another malonyltransferase capable of catalyzing this reaction, such as the MAT gene from *Arabidopsis* described in the Examples. The locus in TAIR is AT3G29590, and the genomic sequence in genbank is NM_113880.

The invention further provides a process for producing Compound **V** (a compound comprising the cation of formula **V** or a neutral or anionic form thereof) from Compound **III** (a compound comprising the cation of formula **III** or a neutral or anionic form thereof) in eukaryotic cells, comprising optionally expressing in said cells proteins, or ORFs or genes encoding them, which allow production of Compound **III** in said cells, and expressing in said cells an acyltransferase defined with reference to **SEQ ID NO: 3** as above, or an ORF or gene encoding it, and a glucosyltransferase as defined with reference to **SEQ ID NO: 4** as above, or an ORF or gene encoding it.

Preferably, Compound **V** may be produced from Compound I (D3G) in eukaryotic cells, comprising expressing in said cells
(i) an acyltransferase, or an ORF or gene encoding it, capable of transferring a p-coumaryol group from p-hydroxycinnamoyl-CoA to D3G to produce Compound **II,** such as the acyltransferase defined above with reference to **SEQ ID NO: 1,**
(ii) a glucosyltransferase, or an ORF or gene encoding it, defined above with reference to **SEQ ID NO: 2,**
(iii) an acyltransferase, or an ORF or gene encoding it, as defined above with reference to **SEQ ID NO: 3,** and
(iv) a glucosyltransferase, or an ORF or gene encoding it, as defined above with reference to **SEQ ID NO: 4.**

The invention also provides a process of producing delphinidin 3-*O*-[2-*O*-(6-*O*-p-coumaroyl)-glucosyl-6-O-p-coumaroyl] glucoside (Compound **IV;** comprising the cation of formula **(IV)** or a neutral form or anionic form thereof) from D3G (comprising the cation of formula (**I**) or a neutral form or anionic form of D3G) in eukaryotic cells, comprising expressing, in said in the eukaryotic cells,
(i) an acyltransferase, or an ORF or gene encoding it, capable of transferring a p-coumaryol group from p-hydroxycinnamoyl-CoA to D3G to produce Compound **II,** such as that defined above with reference to **SEQ ID NO: 1,**
(ii) a glucosyltransferase, or an ORF or gene encoding it, a as defined above with reference to **SEQ ID NO: 2,** and
(iii) an acyltransferase, or an ORF or gene encoding it, as defined above with reference to **SEQ ID NO: 3.**

In the processes described above, the product produced is produced in said eukaryotic cells. Accordingly, the product accumulates in these cells. The products may be isolated from the cells or plants according to generally known methods or as further described below.

The invention also provides a polynucleotide (i.e. a nucleic acid molecule) comprising a coding sequence encoding one or more of the proteins described above. The polynucleotide may be DNA or RNA, but DNA is preferred. Preferably, the polynucleotide contains one or more of the ORF(s) or gene(s) mentioned above. The term ORF (open reading frame) refers to a polynucleotide encoding a protein of the invention (i.e. a coding sequence or CDS). The term "gene" herein refers to a polynucleotide comprising a coding sequence encoding the respective protein and one or more regulatory elements, such as a transcription promoter and transcription terminator as required for expressing the gene in the eukaryotic cells used. The polynucleotide may comprise more than one coding sequence (ORF) or gene, for example for transforming or transfecting a eukaryotic cell with more than one coding sequence or gene. As described in the Examples, the polynucleotide or construct may contain all five genes for expressing proteins catalyzing all five reaction steps shown in **Fig. 1** up to verodelphin, see e.g. construct pAGM89751 shown in **Fig. 4****.** The polynucleotide may comprise further genes for co-expression in eukaryotic cells, e.g. one or more transcription factors, such as Rosea1 and/or Delila, e.g. for promoting production of D3G or precursors upstream of D3G in the biosynthetic pathway leading to D3G. The skilled person understands that the promoters are selected so as to allow expressing the coding sequences or genes in the cell type or plant tissue where expression is desired.

The invention also provides a (recombinant) construct, comprising the polynucleotide of the invention. The invention further provides a vector for transforming or transfecting a eukaryotic cell or plant, said vector comprising the polynucleotide as described above or said construct. In addition to the polynucleotide as described above, the construct and vector may additionally contain genetic elements, such as T-DNA left and right boarders for inserting the polynucleotide into plant cells. The vector may further contain a selectable marker for cloning in bacterial cells. For *Agrobacterium-mediated* transformation or transfection, the vector may be a binary vector comprising T-DNA comprising a polynucleotide of the invention (see further below).

A convenient method for generating polynucleotides and constructs containing multiple genes is Golden Gate (GG) cloning that makes use of type IIS restriction enzymes for restriction and seamless ligation, cf. WO 2008/095927, WO2011154147, Marillonnet, S., & Grützner, R. (2020). Current Protocols in Molecular Biology, 130, e115. doi: 10.1002/cpmb.115.

Alternatively, coding sequences (ORFs) encoding two or more proteins of the invention may be linked by a coding sequence encoding a 2A peptide (also referred to as P2A peptide). The two or more ORFs encoding proteins of the invention can then be expressed as a fusion protein from a single promoter (and using a single terminator). For this purpose, stop codons of the protein-encoding sequences preceding a 2A peptide-encoding sequences should be removed to allow expression of the fusion protein. Ribosome skipping at a sequence in the 2A peptide results in production of two or more distinct proteins, even though there is only one large coding sequence encoding the fusion protein. Such approach makes engineering easier. In one embodiment, the 5 ORFs encoding the 5 proteins of the present invention are fused together by four 2A peptide-encoding sequences. The 2A peptide has the amino acid sequence of SEQ ID NO: 26. Use of the 2A peptide is explained by He et al., Horticulture Research (2020) 7:152; doi.org/10.1038/s41438-020-00390-1. Synthetic polycistronic sequences in eukaryotes, using in particular 2A sequences, are described in Wang et al., Synthetic and Systems Biotechnology 6 (2021) 254-261, doi.org/10.1016/j.synbio.2021.09.003.

In one embodiment, the invention provides a polynucleotide (or nucleic acid molecule), construct or vector comprising:
(i) a coding sequence or gene encoding an acyltransferase capable of transferring a p-coumaryol group from p-hydroxycinnamoyl-CoA to D3G to produce Compound II, such as the acyltransferase defined above with reference to **SEQ ID NO: 1,**
(ii) a coding sequence or gene encoding a glucosyltransferase as defined above with reference to **SEQ ID NO: 2,**
(iii) a coding sequence or gene encoding an acyltransferase as defined above with reference to **SEQ ID NO: 3,**
(iv) a coding sequence or gene encoding a glucosyltransferase as defined above with reference to **SEQ ID NO: 4,** and
(v) a coding sequence or gene encoding a malonyltransferase capable of transferring a malonyl group from malonyl-CoA to Compound **V** to produce Compound **VI**, such as the malonyltransferase defined above with reference to **SEQ ID NO: 5.**

In another embodiment, the invention provides a polynucleotide (or nucleic acid molecule), construct or vector comprising:
(i) optionally one or more coding sequences or genes, expression of which in eukaryotic allows production of Compound **III** in said cells, such as a coding sequence or gene encoding an acyltransferase capable of transferring a p-coumaryol group from p-hydroxycinnamoyl-CoA to D3G to produce Compound II, such as that defined above with reference to **SEQ ID NO: 1,** and a coding sequence or gene encoding a glucosyltransferase defined above with reference to **SEQ ID NO: 2,**
(ii) a coding sequence or gene encoding an acyltransferase defined with reference to **SEQ ID NO: 3** as above and
(iii) a coding sequence or gene encoding a glucosyltransferase as defined with reference to **SEQ ID NO: 4** as above.

In another embodiment, the invention provides a polynucleotide (or nucleic acid molecule), construct or vector comprising:
(i) a coding sequence or gene encoding an acyltransferase capable of transferring a p-coumaryol group from p-hydroxycinnamoyl-CoA to D3G to produce Compound II or a neutral or anionic form thereof, such as the acyltransferase defined above with reference to **SEQ ID NO: 1,**
(ii) a coding sequence or gene encoding a glucosyltransferase a as defined above with reference to **SEQ ID NO: 2,** and
(iii) a coding sequence or gene encoding an acyltransferase as defined above with reference to **SEQ ID NO: 3.**

In the production processes of the invention, one or more ORFs or genes encoding the desired enzyme(s) of the invention are expressed in the eukaryotic cells or in plants. Gene expression is generally known in the art in eukaryotic cells as well as in plants. The processes may be carried out in eukaryotic cells, such as yeast cells or plant cells, or in plants. The eukaryotic cells may be yeast or plants cells. Alternatively, the processes are carried out in plants, such as in entire plants or, preferably, in certain plant tissues, such as petals or fruits. The skilled person understands that promoters are suitably chosen to allow expression of the genes in the desired cell types or plant tissues or organs.

Examples of eukaryotic cells are yeast cells, such as of genus *Saccharomyces* (most preferably *Saccharomyces cerevisiae)* or *Schizosaccharomyces* (e.g. *Schizosaccharomyces pombe), Yarrowia lipolytica,* or *Pichia pastoris.* The yeast cells (or yeast strains) may be edible. Other fungi such as molds, e.g. *Fusarium fujikuroi* could also be used in the invention. Yeast cells are the preferred non-plant eukaryotic cells for practicing the invention. Yeast cells may be transformed according to generally known methods, cf. Borts, Rhona H. (1996) Yeast protocols: Methods in cell and molecular biology. Methods in Molecular Biology (Book 53) Humana Press. Methods for nucleic acid expression in *S*. *pombe,* including vectors, promoters, terminators, transformation methods, integration into a target region e.g. using homologous recombination are known in the prior art, e.g. from EP 2 468 860 A1. Production of anthocyanins such as D3G in yeast is described in [4].

Examples of plants or cells thereof are monocot and dicot (preferably crop) plants, such as from *Solanaceae* and Rosaceae. Examples of Solanaceae are tomato *(Solanum lycopersicum),* potato *(Solanum tuberosum),* roses, and *Nicotiana* species such as N. *tabacum* and *N. benthamiana.* Examples of monocot plants are tulips and orchids. Generally, the plant or cells thereof are from a plant other than *Veronica persica.*

In one general embodiment, verodelphin is produced in large amounts for use as a colorant, such as food colorant. For such purpose, the verodelphin may be produced (and the required ORFs or genes be expressed) in plant fruits in order to produce large amounts thereof. Possible plant fruits are potato tubers or tomatoes.

In another general embodiment, verodelphin is produced in order to alter the color of petals in flowering plants. In this case, the verodelphin may be produced (and the required genes be expressed) in petals of the flowering plants, the petals of which are to be changed in color. Examples of such flowering plants are tulips, orchids and roses.

For producing verodelphin or a compound selected from Compound II, III, IV and V in plants or plant cells, plants or cells thereof may be transiently transfected or stably transformed with the ORFs or genes required for verodelphin product. Both transient transfection and stable transformation of plants for expressing one or more gene of interest is known in the art. The term "transient" or "transient transfection" means that no selection methods are used for selecting cells or plants transfected with the nucleic acid molecule (vector) or with the nucleic acid construct from non-transfected cells or plants using, e.g. a selectable agent and a selectable marker gene capable of detoxifying the selectable agent. As a result, the transfected nucleic acid is generally not stably introduced into plant chromosomal DNA. Instead, transient expression methods make use of the effect of transfection in the very plant cells transfected.

A possible way of achieving transient expression of an ORF or gene of interest is the use of self-replicating (viral) replicons containing a nucleotide sequence encoding said nucleotide sequence of interest. Plant viral expression systems have been described in many publications both for the use of DNA viral vectors and for the use of RNA viral vectors, such as in WO2008028661, WO2006003018, WO2005071090, WO2005049839, WO2006012906, WO02101006 or WO02068664 and many more publications are cited in these documents.

Various methods for introducing a polynucleotide, such as a DNA molecule, into a plant or plant part for transient expression are known. For example, Agrobacteria may be used for transfecting plants with the gene, construct or vector, e.g. by agroinfiltration. A system and method for large scale infiltration of plants by agrobacteria is described in WO2009095183. In another embodiment, plants or plant parts are sprayed with a suspension containing cells of an *Agrobacterium* strain, which is well suitable for large scale applications to many plants such as to plants on a farm field. Such spray transfection processes are described in detail in WO2012/019660. More information on transient expression by *Agrobacterium-mediated* transfection is found in WO 2014/187571, WO 2013/149726 and WO2012/019660, as well as below.

In stable transformation, a polynucleotide is integrated into a chromosome such as a nuclear or plastid chromosome of a plant cell, such that the nucleic acid can be transferred to progeny cell or progeny plants. Integration into a nuclear chromosome is preferred. Selection methods using a selectable marker gene in the recombinant construct or vector allows selecting transformed plant cells or plants out of the large background of non-transformed cells or plants. From transformed cells or tissue (such as callus tissue), transgenic plants containing the nucleic acid can be regenerated using generally known methods. For stable transformation, transient transfection is followed by selection steps using a selectable marker as mentioned above. *Agrobacterium-mediated* gene transfer and vectors therefor are known to the skilled person, e.g. from the references cited above or from textbooks on plant biotechnology such as Slater, Scott and Fowler, Plant Biotechnology, second edition, Oxford University Press, 2008.

The plant or plant cells used for production of verodelphin may, in addition to the ORFs or genes for producing verodelphin, be stably transformed with one or more genes allowing or facilitating production of D3G in said plant or plant cell, such as the transcription factors Rosea1 and/or Delila. Alternatively or additionally, the plant or plant cells may be genetically modified to have a native gene encoding a rhamnosyltransferase silenced or deactivated in order to avoid production of delphinidine-3-O-rutinoside (D3R), see Ref. 15.

Several possibilities exist to provide a eukaryotic cell with the polynucleotide(s) for practicing the processes of the invention. The polynucleotide(s) may, for example, be injected into or be taken up by (for example upon electroporation or PEG mediated transformation) the eukaryotic cell as a mixed solution of one or more polynucleotides of the invention.

Various methods for introducing polynucleotide(s) into plant cells, cells of a plant or a plant are known, and examples are electroporation, PEG (polyethylene glycol) transformation, microinjection, particle bombardment, and the use of viral vectors. Again, these methods are particularly suitable for transiently provided the components to plant cells. However, the preferred method of introducing the polynucleotide(s), construct(s) or vector(s) of the invention into plant cells or cells of a plant is *Agrobacterium-mediated* transformation. *Agrobacterium*-mediated transformation is well-established in the field of plant biotechnology, e.g. from textbooks on plant biotechnology such as Slater, Scott and Fowler, Plant Biotechnology, second edition, Oxford University Press, 2008. It comprises contacting living plant tissue (e.g. leaves or floral tissue) with a suspension of *Agrobacterium* cells containing a Ti-plasmid and a binary vector comprising T-DNA. Entry of the *Agrobacterium* cells into the plant tissue may be facilitated by a pressure difference (e.g. using a needleless syringe, also referred to as "agroinfiltration") or sucking (e.g. vacuum infiltration). Alternatively, plant tissue may be sprayed with a suspension containing *Agrobacterium* cells and optionally an abrasive and a surfactant, e.g. as described in WO2012019660.

For *Agrobacterium-*mediated transfection, a first nucleic acid molecule (or polynucleotide) of the invention may be a plasmid (such as binary vector) containing in its T-DNA a first recombinant construct encoding a first protein or further protein(s) of the invention. A second nucleic acid molecule (or polynucleotide) of the invention may be a plasmid (such as binary vector) containing in its T-DNA a second recombinant construct encoding a second and/or further protein of the invention. However, it is possible to produce a binary vector that encodes in its T-DNA more than one protein of the invention, such as two, three, four, or five proteins of the invention. Optionally, such plasmid may additionally contain a gene encoding a further protein, such as one or more transcription factors. If two or more nucleic acid molecules are used, each type of nucleic acid molecules may be separately introduced as a binary vector in *Agrobacterium* and cultured to produce *Agrobacterium* suspensions. For transformation or transfection, the one, two or more *Agrobacterium* cultures or suspensions, each containing one binary vector, may be mixed and the mixture may be used for transforming plant cells or cells of a plant.

The suspension of agrobacteria may be produced as follows. A nucleic acid molecule or vector may be transformed into the *Agrobacterium* strain and transformed *Agrobacterium* cultures may be grown preferably under application of selective pressure for maintenance of the nucleic acid molecule in question. In one method, the *Agrobacterium* strain to be used in the processes of the invention is then inoculated into a culture medium and grown to a high cell concentration. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water or Agrobacterium infiltration medium may be used. The water may contain a buffer or salts. The water may further contain the surfactant or wetting agent. Alternatively, the concentrated agrobacterial suspensions may be diluted with water, and any additives such as the surfactant and the optional buffer substances are added after or during the dilution process. Separately produced suspensions for co-transfection may then be mixed and the mixed suspension be used for transfecting plant cells or cells of a plant.

The *Agrobacterium* may belong to the species *Agrobacterium tumefaciens* or *Agrobacterium rhizogenes* that are commonly used for plant transformation and transfection and which are known to the skilled person from general knowledge. The *Agrobacterium* strain to be used in the processes of the invention may comprise a nucleic acid molecule (Ti-plasmid or binary vector). The recombinant construct(s) is/are typically present in T-DNA of the plasmid or binary vector for introduction of the polynucleotide or construct into plant cells by the secretory system of *Agrobacterium.* On at least one side or on both sides, the nucleic acid construct(s) is/are flanked by a T-DNA border sequence for allowing transfection of said plant(s) and introduction into plant cells or cells of a plant. Preferably, said construct(s) is/are present in T-DNA and flanked on both sides by T-DNA border sequences. Accordingly, the invention also provides an *Agrobacterium* cell containing a polynucleotide, construct or vector according to the invention.

If plant cells in cell culture are to be transfected, an *Agrobacterium* suspension may be added to the plant cell culture. If selected parts of a plant such as plant leaves are to be transfected, the generally known agroinfiltration may be used, whereby a pressure difference is used to insert the *Agrobacterium* suspension into plant tissue. For example, a needle-less syringe containing the *Agrobacterium* suspension may be used to press an *Agrobacterium* suspension into plant tissue. In another agroinfiltration method, an entire plant or major parts of a plant is dipped upside down into an *Agrobacterium* suspension, a vacuum is applied and then quickly released, whereby an *Agrobacterium* suspension is inserted into plant tissue.

As indicated above and as done in Example 1, it is preferred to transform or transfect into the plant cells or cells of a plant a polynucleotide encoding all desired ORFs or genes.

Verodelphin produced in plant tissue such as petals or fruits or in plant cells or other eukaryotic cells may be isolated and purified from such tissues or cells. The verodelphin may be extracted as described for compound 1 in [12]. The solvent may then be evaporated to obtain verodelphin in dry form.

### EXAMPLES

### Example 1: Verodelphin biosynthesis by transient expression in Nicotiana benthamiana leaves

Expression constructs for the 5 genes of the pathway, (pAGM70427 (VpAT1), pAGM71774 (UGT94F1), pAGM79235 (Vp AT2), pAGM79247 (Vp 5GT), pAGM70438 (Vp MAT)), and the two constructs needed for inducing D3G biosynthesis in *Nicotiana benthamiana* (constructs pAGM45244 for expression of Rosea1 (Addgene Plasmid. #219509) and pAGM10775 for expression of *Arabidopsis* ANS (Addgene Plasmid #219510)) were transformed in *Agrobacterium* strain GV3101:pMP90. The resulting *Agrobacterium* strains were infiltrated in leaves of the *Nicotiana benthamiana* strain Nb 29-2 lacking rhamnosyltransferase activity as previously described [15]. An extract of the infiltrated leaf area performed 5 days after infiltration was analyzed by LCMS as previously described [15]. Verodelphin was produced at high level in the infiltrated leaf (Fig. 2).

Anthocyanin biosynthetic enzymes from other species that catalyze similar enzymatic reactions as those described here may be able to replace some of the *Veronica persica* enzymes for biosynthesis of verodelphin. Examples of such enzymes include *Arabidopsis* At3AT1 (AT1G03940), At5GT (At4g14090) and At5MAT (At3g29590), which may replace the *Veronica* enzymes VpAT1, Vp5GT and VpMAT, respectively, for biosynthesis of verodelphin. The *Arabidopsis* and *Veronica* enzymes are quite divergent at the protein level, with amino acid identities of 31%, 44% and 34% for the pairs At3AT1/VpAt1, At5GT/Vp5Gt and At5MAT/VpMAT, respectively. Nevertheless, we found that substitution of the *Veronica* enzymes VpAt1 and VpMAT by the *Arabidopsis* enzymes in the transient expression assay also resulted in biosynthesis of verodelphin (Fig. 6). In contrast, replacement of Vp5GT by the Arabidopsis enzyme did not lead to efficient biosynthesis of verodelphin, but only to a minor amount of this anthocyanin.

### Example 2: Verodelphin biosynthesis in tomato fruit

Tomato plants do not normally produce anthocyanins in fruits. However, anthocyanins can be produced in fruits by inducing expression of the native tomato anthocyanin biosynthetic pathway using the *Antirrhinum majus* transcription factors Rosea1 and Delila using the fruit-specific promoter E8 [1]. The anthocyanins that are produced are the same that are normally produced in tomato stem and leaves and consist of petunidin-3-O-[4-(trans-p-coumaroyl)-rutinoside]-5-O-β-D-glucoside (Fig. 3). The structures of this anthocyanin and of verodelphin indicate a conflict for the biosynthesis of verodelphin in tomato. More specifically, the step consisting of addition of a coumaroyl residue on the glucose of D3G for verodelphin biosynthesis will compete with addition of a rhamnose at the same position for the tomato anthocyanin. Therefore, it is necessary to remove the rhamnosyltransferase activity of tomato. This can be done by mutagenesis using CRISPR or by silencing (see below).

Tomatoes producing verodelphin can be generated by transforming a construct containing all anthocyanin biosynthetic genes needed, as shown in construct pAGM89751 **(****Fig. 4****).** This construct contains a NPTII selectable marker for selection of transgenic plants on kanamycin-containing media, two transcription units for expression of the transcription factors Rosea1 and Delila for inducing anthocyanin biosynthesis (homologous transcription factors from other species could also be used), and the 5 *Veronica persica* genes for verodelphin biosynthesis. For expression in fruits, Delila1 and Rosea are expressed from the tomato E8 promoter [16]. The five veronica biosynthesis genes are placed under control of promoters of anthocyanin biosynthesis genes that are transcriptionally activated by Rosea1 and Delila (or their homologues from other species). Examples of anthocyanin biosynthesis promoters include the promoters of the *Arabidopsis* genes tt19 (AT5G17220), F3H (AT5G17220), UGT79B1 (AT5G54060), UGT75C1 (AT4G14090) and At5MAT (AT3G29590). The constructs were made using the modular cloning system MoClo [17]. All gene coding sequences were first "domesticated" (meaning that restrictions sites for Bsal and Bpil were removed from internal sequences using silent mutations) and cloned as level 0 modules. This enables the assembly of multigene constructs using the MoClo system. The coding sequences of these five genes in construct pAGM89751 consist of the cDNA sequences of the native genes. However, they could also consist of codon-optimized sequences, or, for VpAT1 and VpAT2, of the native genomic coding sequences containing introns. In pAGM89751, VpAT1 and Vp5MAT could also be replaced by the coding sequences of the genes from Arabidopsis thaliana At3AT1 (AT1G03940) and At5MAT (AT3G29590), respectively, as these *Arabidopsis* genes are functionally equivalent to the *Veronica* genes, or also from homologous genes from other species. The terminators of the transcription units present in the construct were selected to be all different from each other to avoid recombination between repeated sequences. These terminators can consist of any functional plant terminator, including the *Agrobacterium* Nos, Mas or Ocs terminators, the viral 35S terminator, the *Arabidopsis* C4H (AT2G30490), PAL (AT2G37040) or TT19 (At5g17220) terminators, and the pea E9 terminator.

The resulting transgenic plants are expected to produce a mix of verodelphin and of the native tomato anthocyanins. To avoid biosynthesis of tomato native anthocyanins, the tomato rhamnosyltransferase gene can be inactivated. Two solutions are possible.

The first strategy is to inactivate the tomato rhamnosyltransferase gene (Genbank LOC101244316) using CRISPR mutagenesis. For this purpose, a CRISPR Cas9 construct was made. This construct contains a selectable marker for transformation, a Cas9 gene and a guide RNA designed to introduce mutations in the tomato RT gene using guide RNA sequences CAGGAGAATTAGAAGAACGA **(SEQ ID NO: 14).** An additional guide RNA could be included in the construct to inactivate the tomato methyltransferase gene (Solyc09g082660) using guide RNA ATGGGTGTGCCTCGAGATGA **(SEQ ID NO: 15).** The construct could also, optionally, contain a guide RNA to mutate the phytoene synthase 1 gene (Solyc03g031860) to prevent lycopene biosynthesis in the tomato fruit, to achieve a bluer color of the fruit after crossing with the transgenic plant containing the *Veronica* anthocyanin biosynthetic genes.

Transgenic plants transformed with construct pAGM89751 should then be crossed with CRISPR lines containing construct pAGM83553. The resulting plants that have a knockout phenotype for the tomato RT and OMT should produce only verodelphin and not the native tomato anthocyanin. In the next generation, the Cas9 construct could be eliminated by segregation, keeping only sequences from pAGM89751 and the mutations in the RT and OMT, resulting in tomato plants with a bluish color and blue juice.

An alternative strategy for inactivating the rhamnosyltransferase activity of tomato could consist of adding a silencing hairpin construct to sequences present in pAGM89751 resulting in pAGM89752. The silencing sequence consists of two segments of the tomato rhamnosyltransferase gene (Genbank LOC101244316) in opposite orientations separated by spacer sequence (SEQ ID NO: 11). This silencing hairpin sequence is placed under control of the E8 promoter. Tomato plants transformed with pAGM89752 should directly produce verodelphin and should exhibit a blue color. An alternative to placing the silencing construct in the same construct as all other genes needed is to make a separate construct (pAGM90825) containing a selectable marker and the hairpin sequences under control of the E8 promoter. This construct should be transformed into wildtype tomato, and the resulting transformants crossed to pAGM89751 transformants described above.

In the examples described above, the tomatoes produced should have a blue juice, but the color of the fruit should not be completely blue due to the underlying presence of carotenoids. The use of a tomato line mutant for Psy1 *(Solanum lycopersicum* gene Solyc03g031860.2) for transformation would result in fruits with a bluer appearance, as Psy1 mutant plants exhibit lower levels of carotenoids in fruits. Fruits of Psy1 mutant plants however still contain a residual amount of beta carotene due to the expression of the tomato Psy2 gene [18]. This gene cannot be inactivated by mutagenesis, as this mutation would be lethal for plants. It should be possible to express in the fruit a carotenoid cleavage enzyme to cleave residual amounts of beta carotene, for example using the *Medicago truncatula* gene MtCCD1 (GenBank FM204879) [19] fused to a transit peptide sequence for expression in the chloroplast, or the tomato gene Le CCD1b (Genbank AY576002) fused to a chloroplast transit peptide sequence.

It has been shown that expression of the *Arabidopsis* MYB12 transcription factor from the E8 promoter can lead to increased yield of anthocyanins in the fruit. It is therefore possible to generate transgenic plants as described above but expressing, in addition, a transcription factor such as *Arabidopsis* MYB12 for increased production of blue anthocyanins in tomato fruits.

In addition, it may be possible to increase the fruit anthocyanin content by inactivating repressors of anthocyanin biosynthesis such as *SIMYBATV* (Cao et al., 2017) using either CRISPR mutagenesis or RNAi silencing.

### Example 3: Determination of the order to steps leading from D3G to verodelphin

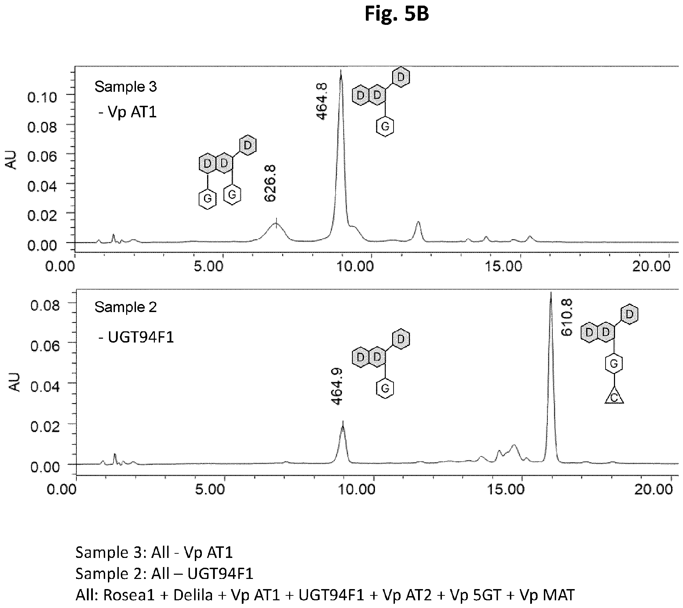

To determine the sequential role of each gene in the synthesis of verodelphin, we conducted a series of infiltrations **(****Fig. 5A****).** As a control, we infiltrated all five genes responsible for verodelphin production into *N. benthamiana* leaves. In separate experiments, we individually removed one of the five genes to assess its impact **(****Fig. 5B-****D).** We analyzed the resulting anthocyanins using LC-MS for each infiltration. When Vp At1 was removed, the major peak observed was delphinidin 3-glucoside **(****Fig. 5B****),** indicating that Vp At1 is the initial enzyme in the pathway. The second enzyme to act is UGT94F1, as the main peak produced is compound II **(****Fig. 5B****).** Vp At2 is the third enzyme acting, as compound III is the main peak produced when this enzyme is absent **(****Fig. 5B****).** Vp 5GT and Vp MAT were nevertheless able to act on this substrate and generate some amount of compounds lacking the acyl group added by Vp At2. Vp 5Gt and Vp MAT are the last two enzymes of the biosynthetic pathway to verodelphin.

Anthocyanin A11 presents some similarity with verodelphin, even though it has a cyanidin backbone rather than a delphinidin backbone **(****Fig. 6A****).** In particular, 3 enzymatic reactions perform the addition of sugar or acyl groups at equivalent positions in the anthocyanin. These consist of At 3AT1 (AT1G03940), At 5GT (UGT75C1, At4g14090) and At 5MAT (AT3G29590) (Fig. 6A). To test whether these enzymes could functionally substitute for equivalent *Veronica persica* enzymes, we performed infiltrations where one construct was replaced by a construct containing an *Arabidopsis* gene **(****Fig. 6B****).** These experiments show that At 3AT1 could replace Vp At1 and lead to biosynthesis of verodelphin (**Fig. 6C**). Also, *At* 5MAT can replace *Vp* MAT for biosynthesis of verodelphin **(****Fig. 6E****).** However, use of *At* 5GT instead of *Vp* 5GT led to only a minor amount of verodelphin (**Fig. 6D**). The main peak produced consists of a compound lacking the acyl group normally catalyzed by use of *Vp* At2. This is probably because incorporation of the sinapoyl group in A11 takes place after addition of the glucose at position 5 by *At* 5GT. Therefore, At 5GT normally acts on a substrate that does not have an acyl group at the equivalent position catalyzed by *Vp* At2.

### NUCLEOTIDE AND AMINO ACID SEQUENCES

SEQ ID NO: 1: Vp AT1 amino acid sequence 
MPTTPTISVTIHEHCPIAPPAGDDQVSELNLPLSYFDVSWMNFPPASCLLFYRFPCSSVHFH
ETVVLNLKKSLSQTLKHYLPLAGNLLVPLSLDMPEFRYVKGDTLRFTIAEANVDFDFDYWTG
NQARDAAQFHAFVPDLPEPKIDTDTGFRIMPVQSVQVTLLPGTGICFGFASDHAALDARSLV
MFVKCWSSLTKLGKEDGELLAKNGLLPLFDRSVIIDPSNERANIFWDQMKQKPRFPKKLPVD
VVRQTFVLQKSDLQKLRNSALAKRPGLVHLSSFTILSAYIWTCHVKAATKLGEEVNENEPEY
YVIAMDGRHRLDPPVPAEYFGNCITPAVVASTHGQLKGEDGFCTAVELIGETISKLNSTEEF
MKNNADDWLGKIKHVFRFQRNLTLAGSPKFDLYDTDFGWGTPNKYEVISIDGDNSLSLCKSR
EFEGGLELGLSLPKSKVDAFATFFVEGLKL
**SEQ** ID NO: 2 UGT94F1 amino acid sequence 
MEKEEAKMFKILMFPWLAHGHIFPFLELAKTLSKRNFTIHFCST
AINLDSIKSNLANDPSVLDDSIKLLELEIESPELPPELHTTKNLPPHQFPLLIKDFEN
SKSSFFSIFDTLKPDMLIYDVFNPWAAKHALSHGSPSVWFMASGATICSFHYHQHLHK
TGSLVPYEGVDFGEIKRHISPNTKGADFGGFILGSLNSSSEIILLKTSKELEKKYIDY
LSFLCRKQIIPTGLLIANSDEKDEPEIMQWLDEKSERSTVYISFGSECFLSKEQIEEV
AKGLELSNVNFIWIIRFPEGKNSMTVENALPEGFLERVKGRGMVIWKFAPQTRILAHK
SIGGFVSHCGWSSITESVYFGVPIIAMPMKFEQVVNGVVVVEVGVGVEVEKDGSGQYL
GEEVAKALDKVFGDNEFSKEVRYRASNLSDKIRENEEQEEDKVAEQLMSLCAKNKLQK
CD
**SEQ** ID NO: 3 Vp AT2 aminoacid sequence 
MATTPAKRITIHKHCRVAPPVGDGEVVEQVLKTTYMDMPWVYFNPIQCVLFFRFPCSKVHFD
ETIIPNLKQSLSQALKHYLIVAGNVYIPLESGMPELRYVSGDSVSFIIAESDGDIEFDYLTG
NHPRDACQFYEFVPYLPELKIDPDNEFKTMPVLSAQVTLFPDKGLSLGWATHHVAGDATSFI
RFLKAWSSLANVGKDVDTYDFLAKNSLHSLFDRSVIADPSGRRANIYWNQIKKSGRIAPPPK
LPTDKLRQTFGLTKSDMQKLRNSALAKKPWLVHLSTYTIVSAYLWSCFVKIASKIGEEVDAQ
EPEFFLMSVDARARLEPPVPLEYYGNCIVPAVVGSTHGELKGDDGFSNAVELIGDVISKQIN
NKEEFMKSADNQLERIRPIFGKRLLTVNGLPNFDMYGTDFGFGKTYKYEVLSIDGANCMSLC
KSRDSEGGSEIGLTLPKPKLDAFASIFSEGLN
**SEQ** ID NO: 4 Vp 5GT amino acid sequence 
MAGPRILVATFPAQGHINPSLQFAKRLIKMGSEVTFLISDYASHRIAGTTGGVYPKGLTVVS
IPDGFGQKPADGDEKKHMEQMKSRGLKAVRDTLLEGVEQGRRFSCLVYSHLFAAAAEVARET
KTPSALLWIEPATVLDIYYFYFNGYKDEIDKGDEIKLPGLPLLAQRDLPTFLLPSMPERFRL
FMKEKLDILDAAEKPKVLINTFDVLEPDAINAIHKYEMVGIGPLIPSAYLDGLDPSDRSFGG
DLVKKSEDSYLQWLSTNSESSVVYVSFGSGVMLPLPQLEEIAEALLKCGRPFLWVVRVNEDN
ERPEVEKLSCFKELEKLGKILTWCSQLEVLTHHSLGCFVTHCGWNSALESIALGVPVVAFPQ
FFDQGTNAKLIEDVWRTGVRVNRNEDKCVGRDEISRCIEMVMDGGERSQEMRENARKWKGLA
RESMEENGSSNKNLKAFLDELGDDEVIAVSQ
**SEQ ID NO: 5** Vp MAT amino acid sequence 
MTISTNKVTVHAHCGVAPPVEMTEQRLSLTYFDMTWVHFHPIQRLIFYKFPCSTVQFYETIV
PNLKESLSQTLKHFLPLAGNLYLPILNNPGFPEFRYVPGDTVSVTIAESSEDFDFDYWTGNH
ARDADEFYGFVPELSEPKIDSDSGLKIIPLVAVQVTVFPGTGISLGFTNNHAIGDANSIVRF
IKSWATVSKLGKDADDAFLTKNELLPFFDRSVIKDPSQKRANIFWTEMKKRLSFVPPPSFPT
NKFRETFILQQSDLQRLRIAAITKKPGLDYLSSFTISVAYVWSCIVKTASKSGEEVDENAAE
CILFSADARPRMDPPAPASYFGNCLVGVVTESTHGKLKGEEGFCTAVELIGELITKQVNNKD
ELLKDADEWLVKYKPLFGKRVFGVSGSPKFDLYDTDFGWGKAHKCESISIDKDSSLSICKSR
EFKGGLEIGMSLSKLKLDTFAAVFANGLEK
**SEQ** ID NO: 6, Vp AT1 CDS 
ATGCCAACAACACCTACAATAAGTGTCACAATACACGAACACTGCCCTATCGCACCGCCTGC
TGGCGACGACCAAGTGTCGGAGTTAAACCTTCCTCTGTCATACTTCGACGTGTCATGGATGA
ACTTTCCGCCCGCTTCGTGTCTCCTTTTCTACCGGTTCCCCTGTTCCAGCGTTCATTTCCAT
GAAACCGTAGTTCTTAATCTGAAAAAATCGTTATCTCAAACTCTTAAACACTACCTTCCCCT
CGCAGGCAACTTATTAGTTCCGTTGAGTTTAGACATGCCTGAGTTTCGCTATGTCAAGGGAG
ACACTCTTCGTTTCACAATTGCAGAGGCAAATGTTGATTTTGATTTCGACTACTGGACAGGA
AATCAAGCTCGTGATGCAGCTCAATTCCACGCTTTCGTGCCTGATTTACCCGAACCCAAGAT
TGATACTGACACAGGTTTCAGAATAATGCCTGTCCAATCAGTACAAGTGACGCTTTTGCCAG
GAACAGGGATATGTTTTGGATTTGCCAGTGATCATGCTGCTCTGGATGCTCGTTCACTTGTT
ATGTTTGTCAAGTGTTGGAGTTCACTAACCAAGCTTGGAAAGGAAGATGGTGAATTACTAGC
CAAAAATGGTTTACTTCCACTGTTTGACAGATCAGTGATCATAGATCCATCCAATGAACGAG
CAAATATATTTTGGGATCAAATGAAGCAAAAGCCTAGGTTCCCGAAAAAGTTGCCTGTGGAT
GTTGTTAGGCAAACATTTGTGTTACAGAAAAGTGATCTACAAAAACTCAGGAACTCAGCGTT
AGCTAAACGGCCAGGACTAGTTCATTTATCTTCTTTCACTATTCTGTCGGCTTACATTTGGA
CTTGCCATGTAAAAGCCGCAACAAAACTTGGTGAAGAGGTCAATGAAAACGAACCAGAATAC
TATGTCATTGCCATGGATGGTAGACATCGTCTGGATCCACCAGTTCCTGCAGAATATTTCGG
CAATTGCATAACTCCAGCAGTGGTAGCATCAACACACGGACAGCTGAAAGGAGAAGATGGTT
TTTGCACCGCGGTTGAGTTAATAGGCGAGACTATAAGTAAACTTAACAGCACAGAAGAGTTC
ATGAAAAATAATGCTGATGACTGGCTAGGGAAGATCAAGCATGTATTTAGGTTCCAAAGGAA
CTTAACACTAGCTGGATCACCGAAATTCGACCTGTATGATACTGATTTCGGGTGGGGAACAC
CAAACAAATACGAAGTGATTTCGATTGATGGAGATAACTCTCTGTCTCTCTGTAAATCAAGA
GAGTTTGAAGGAGGATTGGAACTTGGTTTATCTCTGCCTAAGTCTAAAGTGGACGCTTTCGC
TACTTTCTTCGTGGAGGGTCTGAAACTAtaa
**SEQ ID NO: 7** UGT94F1 coding sequence 
atggagaaag aagaagcaaa aatgttcaaa attctcatgt ttccatggtt agctcatgga
cacattttcc ctttccttga acttgctaaa acactctcca aaagaaactt taccatacac
ttttgttcca ctgccattaa tcttgattca atcaagtcca accttgctaa tgatccatct
gttcttgatg attcaataaa gctactcgaa ctcgagatcg aatctcctga actccctcct
gaactccaca caactaagaa cttaccacca catcaatttc ctctcttgat aaaagacttt
gaaaactcaa aatcaagttt cttctccatt ttcgatactt taaagcccga tatgctgatt
tacgatgttt tcaatccgtg ggcagccaaa cacgcgttat ctcacggttc gccttctgtt
tggttcatgg catcaggtgc tactatatgt tcttttcatt atcatcagca tttgcacaaa
actggttcgc ttgttcctta tgagggagtt gatttcggcg agatcaagcg tcacattagt
ccaaacacta aaggcgctga tttcggagga tttatattgg gaagtttgaa ttcgtcttcc
gaaatcatct tgctaaagac ctcaaaagaa cttgaaaaga aatacattga ttatctatcg
tttttatgca ggaaacagat tatacctact ggtctactga ttgcaaattc tgatgagaaa
gatgagccag aaatcatgca atggttagac gaaaaaagtg aacgttcgac ggtctacata
tcttttggta gcgagtgctt cttgtcgaaa gaacagatcg aagaggtggc caaagggttg
 
gaactaagta atgtgaattt tatatggatc ataagattcc ctgaaggaaa gaatagtatg
actgttgaaa acgcattgcc tgaaggattc ttagaaaggg tgaaagggcg agggatggtg
atttggaaat tcgcgccaca aacgagaata ttggcacata aaagtattgg tggtttcgtg
agtcactgcg ggtggagttc tataactgaa agcgtgtatt ttggagtacc gattatagcc
atgcctatga aatttgagca agtggtaaat ggtgttgttg tggtggaggt gggcgttgga
gtagaggtcg aaaaggacgg gagtggtcag tatttgggag aagaggtggc aaaagcatta
gataaggtgt ttggggataa tgagtttagt aaagaggtga gatacagagc cagtaattta
agtgacaaaa ttcgcgagaa tgaagaacaa gaagaggata aagtagcaga gcagctgatg
agcctatgtg cgaagaacaa gttgcagaaa tgtgattga
**SEQ ID NO: 8** Vp AT2 CDS 
ATGGCTACTACACCCGCAAAGCGTATAACAATACACAAGCACTGCCGCGTTGCACCACCAGT
TGGTGATGGCGAAGTGGTAGAACAAGTCCTTAAGACGACTTACATGGACATGCCATGGGTCT
ACTTTAATCCCATCCAATGTGTACTCTTCTTCCGGTTCCCCTGTTCCAAAGTTCATTTTGAC
GAAACCATAATCCCGAATCTCAAACAATCTCTCTCTCAAGCACTCAAACATTACCTTATCGT
AGCTGGCAATGTATACATTCCTTTGGAATCCGGCATGCCGGAATTACGCTATGTTTCTGGCG
ACTCTGTTTCTTTCATAATAGCTGAGTCAGATGGTGACATTGAATTCGACTACTTGACAGGG
AACCATCCTCGTGATGCCTGTCAATTCTACGAGTTTGTGCCTTACTTACCTGAACTAAAAAT
AGATCCAGATAATGAGTTCAAGACAATGCCCGTCTTGTCCGCCCAGGTGACTCTCTTCCCAG
ACAAAGGATTAAGTCTTGGCTGGGCCACCCATCACGTTGCTGGAGATGCTACTTCATTCATC
AGATTTCTCAAGGCCTGGAGTTCACTAGCCAACGTTGGGAAAGATGTTGATACTTATGACTT
CTTAGCTAAAAATTCCTTACATTCACTTTTCGATAGATCCGTGATTGCAGATCCATCTGGGC
GGCGAGCAAATATTTATTGGAACCAAATAAAAAAGTCGGGCAGGATAGCTCCCCCACCAAAG
TTGCCTACTGATAAACTTAGGCAAACATTTGGTCTAACAAAAAGTGACATGCAAAAACTCAG
GAACTCAGCGTTAGCTAAAAAGCCGTGGTTAGTCCACTTGTCAACATATACTATCGTTTCTG
CATACCTTTGGAGTTGCTTTGTGAAAATTGCATCAAAAATTGGGGAAGAGGTAGATGCACAG
GAGCCTGAATTCTTTCTCATGTCCGTGGATGCTCGGGCACGTCTGGAACCACCTGTTCCATT
AGAGTATTACGGAAATTGCATAGTTCCAGCAGTGGTAGGATCAACTCACGGAGAATTGAAAG
GAGATGATGGATTTTCCAATGCGGTCGAGTTAATAGGGGATGTTATTAGCAAACAGATAAAT
AACAAGGAGGAATTCATGAAAAGTGCTGATAACCAGCTAGAGAGAATAAGGCCTATATTTGG
CAAAAGGCTTTTAACAGTCAACGGATTGCCAAACTTCGACATGTATGGTACAGACTTCGGGT
TTGGTAAAACATACAAGTACGAAGTGCTTTCCATCGATGGGGCAAACTGCATGTCTCTTTGT
AAGTCAAGAGATTCCGAGGGAGGGTCAGAAATTGGTTTAACTCTGCCTAAACCGAAACTGGA
TGCTTTTGCGTCTATCTTCTCCGAAGGACTAAACTAg
**SEQ ID NO: 9** Vp 5GT CDS 
ATGGCGGGACCTCGAATCTTAGTAGCAACATTTCCAGCACAAGGCCATATCAACCCATCCCT
CCAGTTCGCCAAGAGACTCATAAAAATGGGAAGTGAAGTCACATTTCTCATAAGCGACTATG
CATCGCACCGCATTGCAGGAACTACTGGCGGTGTATATCCAAAGGGACTGACAGTCGTGTCA
ATTCCTGATGGTTTTGGACAAAAACCGGCAGATGGTGATGAAAAGAAACATATGGAGCAGAT
GAAAAGTCGCGGGTTAAAGGCCGTTCGGGATACTCTTCTTGAGGGGGTAGAGCAAGGCCGAC
GATTCTCTTGCCTTGTCTACTCTCACCTCTTTGCTGCGGCAGCTGAAGTAGCACGTGAGACT
AAAACCCCGAGTGCGCTTCTCTGGATTGAACCAGCCACCGTGTTGGATATATACTATTTCTA
TTTCAATGGGTATAAGGATGAAATTGACAAGGGTGATGAAATTAAGCTTCCTGGTCTGCCTC
TATTGGCCCAACGCGATCTTCCAACGTTTCTACTTCCTTCTATGCCCGAGAGGTTTCGGTTA
TTTATGAAGGAGAAGTTGGATATATTGGATGCGGCCGAGAAACCCAAGGTGTTGATTAATAC
ATTTGATGTATTGGAGCCTGATGCAATCAATGCCATTCATAAGTACGAGATGGTTGGAATCG
GACCCTTGATTCCTTCCGCGTACTTGGACGGTCTAGACCCCTCGGATAGATCATTCGGGGGA
GATCTTGTTAAAAAATCAGAGGATAGTTACCTCCAATGGCTGAGCACTAACTCCGAGTCGTC
AGTAGTGTATGTGTCTTTCGGGAGCGGGGTGATGTTGCCTTTGCCACAACTCGAGGAGATAG
 
CAGAAGCGCTGTTAAAATGTGGCAGACCGTTTTTGTGGGTGGTACGAGTGAACGAGGATAAT
GAACGTCCGGAGGTTGAGAAATTGAGCTGTTTCAAAGAGCTTGAAAAACTTGGTAAAATATT
GACATGGTGCTCGCAGTTGGAGGTTCTAACACACCATTCATTGGGATGTTTTGTTACTCACT
GCGGTTGGAACTCTGCACTTGAGAGCATAGCGCTTGGTGTCCCAGTTGTGGCCTTTCCACAA
TTTTTTGATCAGGGGACGAATGCCAAGTTAATAGAAGATGTGTGGAGAACCGGAGTTCGTGT
GAACAGGAATGAAGATAAATGTGTTGGCAGAGACGAGATTAGTAGATGCATTGAGATGGTGA
TGGATGGTGGCGAAAGAAGCCAAGAAATGAGAGAAAATGCAAGGAAATGGAAGGGATTGGCA
AGGGAATCCATGGAAGAAAATGGATCTTCAAATAAGAACCTAAAGGCTTTTCTTGATGAGCT
TGGAGATGATGAAGTCATTGCAGTTTCTCAGTAG
**SEQ ID NO: 10** Vp MAT CDS 
ATGACTATCTCCACAAACAAAGTAACTGTCCACGCGCACTGTGGCGTTGCGCCGCCAGTAGA
GATGACAGAGCAACGCCTTTCGCTGACATACTTCGACATGACATGGGTGCATTTTCATCCAA
TCCAGCGTCTTATCTTCTACAAATTCCCTTGTTCCACTGTTCAGTTTTATGAAACCATAGTC
CCAAATCTCAAAGAATCTCTTTCTCAAACTCTCAAACACTTCCTTCCCTTAGCAGGAAACTT
GTACCTTCCAATACTAAACAATCCAGGATTTCCTGAATTTCGATACGTTCCTGGAGACACTG
TTTCTGTCACGATTGCAGAGTCAAGTGAAGATTTTGATTTCGACTACTGGACTGGAAACCAC
GCTCGTGATGCTGATGAATTCTATGGCTTTGTTCCTGAATTATCTGAACCAAAGATAGATTC
TGATTCCGGCTTAAAAATAATCCCACTGGTAGCCGTTCAAGTGACGGTTTTTCCAGGAACTG
GGATTTCTCTTGGATTTACCAACAATCATGCTATTGGGGATGCGAATTCGATTGTGAGATTC
ATTAAGTCGTGGGCTACGGTTTCCAAGCTCGGGAAAGATGCCGATGATGCATTTCTAACTAA
AAATGAGCTACTTCCATTTTTCGACAGGTCAGTGATCAAAGATCCATCTCAAAAGCGTGCGA
ACATATTTTGGACTGAAATGAAGAAACGCCTAAGTTTTGTCCCACCTCCCAGTTTTCCTACT
AATAAATTCAGGGAAACTTTCATTCTCCAACAAAGTGATCTGCAGAGACTCAGAATCGCTGC
GATAACCAAGAAACCGGGGTTAGACTACTTATCATCGTTTACTATTTCAGTTGCTTATGTTT
GGAGTTGCATCGTTAAAACTGCATCTAAAAGTGGAGAAGAGGTCGATGAAAATGCGGCCGAA
TGCATTCTCTTCTCAGCGGATGCACGACCTAGGATGGACCCTCCAGCCCCTGCTTCTTACTT
TGGCAACTGCTTAGTTGGGGTTGTGACAGAATCAACACATGGAAAGCTAAAAGGAGAAGAAG
GATTTTGCACCGCGGTTGAGTTGATAGGCGAGCTTATCACTAAACAGGTTAACAACAAGGAT
GAGTTGTTGAAAGATGCAGATGAGTGGCTAGTGAAGTACAAGCCTTTGTTTGGAAAACGAGT
ATTTGGGGTCAGTGGATCGCCAAAATTCGACCTGTATGATACAGACTTTGGCTGGGGAAAAG
CGCACAAGTGCGAGTCGATTTCCATTGATAAGGATAGTTCATTGTCAATTTGTAAGTCGAGA
GAATTCAAGGGAGGATTGGAGATTGGTATGTCTTTGTCTAAGCTGAAACTGGACACTTTTGC
TGCTGTTTTCGCTAATGGGTTGGAGAAGtaa
**SEQ ID NO: 11** RT hairpin (spacer in italics) 
ATGCTAACAAGCTCTCCATTCATGGTGTTAAAGTTTCATTTTTCACTGCATCTGGCAATGCA
AACAGAGTGAAATCTATGTTGAACTCTGCTCCAACTACTAGTATCGTCCCTCTCACTCTTCC
GCAAGTCGAAGGTCTACCTCCTGGTGCTGAAAGTACTGCTGAATTGACACCAGTAACTGCTG
AACTTCTCAAAGTTGCTTTAGACCAAATGCAACCACAAATCAAGTCTTTACTTTCCAATCTC
AGACCCCATTTTGTTCTTTTCGACTTTGCTCAAGATTGGCTCCCTAAAATGGCTGATGAATT
GGGGATCAAATCTGTTTATTACTCTGTTTTTGTTGCACTCTCCACTGCTTTTCTTACTTGTC
CTGCTAGAGTCCCTCAACCCAAAAATTGTCCATCTCTTGAGGACATGAAAAAACCTCCACCT
GCATTTCCTCACACCTCTGTTACCTCAGTCAAAACCTTTGAAGCTCAAGATTTTCTATACAT
TTTCAAGAGTTTCCATGGAGGTCCTACAGAGGT
AAATCCCCAGATGAACATGGCATCGTGGTGATTGATGAAACTGCTGCTGTCGGCTTTAACCT
CTCTTTAGGCATTGGTTTCGAAGCGGGCAACAAGCCGAAAGAACTGTACAGCGAAGAGGCAG
TCAACGGGGAAACTCAGCAAGCGCACTTACAGGCGATTAAAGAGCTGATAGCGCGTGACAAA
AACCACCCAAGCGTGGTGATGTGGAGTATTGCCAACGAACCGGATACCCGTCCGCAAGGTGC
 
ACGGGAATATTTCGCGCCACTGGCGGAAGCAACGCGTAAACTCGACCCGACGCGTCCGATCA
CCTGCGTCAATGTAATGTTCTGCGACGCTCACACCGATACCATCAGCGATCTCTTTGATGGG
CAGGCTGTAGGACCTCCATGGAAACTCTTGAAAATGTATAGAAAATCTTGAGCTTCAAAGGT
TTTGACTGAGGTAACAGAGGTGTGAGGAAATGCAGGTGGAGGTTTTTTCATGTCCTCAAGAG
ATGGACAATTTTTGGGTTGAGGGACTCTAGCAGGACAAGTAAGAAAAGCAGTGGAGAGTGCA
ACAAAAACAGAGTAATAAACAGATTTGATCCCCAATTCATCAGCCATTTTAGGGAGCCAATC
TTGAGCAAAGTCGAAAAGAACAAAATGGGGTCTGAGATTGGAAAGTAAAGACTTGATTTGTG
GTTGCATTTGGTCTAAAGCAACTTTGAGAAGTTCAGCAGTTACTGGTGTCAATTCAGCAGTA
CTTTCAGCACCAGGAGGTAGACCTTCGACTTGCGGAAGAGTGAGAGGGACGATACTAGTAGT
TGGAGCAGAGTTCAACATAGATTTCACTCTGTTTGCATTGCCAGATGCAGTGAAAAATGAAA
CTTTAACACCATGAATGGAGAGCTTGTTAG
**SEQ ID NO: 12** Sequence of the target site 61 (SI RT); the underlined triplet is the PAM CAGGAGAATTAGAAGAACGA TGG
**SEQ ID NO: 13** Sequence at the target site 62 (SI OMT); the underlined triplet is the PAM ATGGGTGTGCCTCGAGATGA AGG
**SEQ ID NO: 14** Guide RNA 61
   CAGGAGAATTAGAAGAACGA
**SEQ ID NO: 15** Guide RNA 62
   ATGGGTGTGCCTCGAGATGA
**SEQ ID NO: 16 to 25:** primers used for amplification of the 5 genes Vp AT1, UGT94F1, Vp AT2, Vp 5Gt and Vp MAT
**SEQ ID NO: 26 Amino acid sequence of the 2A peptide** GSGATNFSLLKQAGDVEENPGP
**SEQ ID NO: 27 Nucleotide** sequence of the T-DNA region of pAGM89751 (only in electronic sequence listing)

### REFERENCES

1. Butelli E, Titta L, Giorgio M, Mock HP, Matros A, Peterek S, Schijlen EG, Hall RD, Bovy AG, Luo J, Martin C (2008) Enrichment of tomato fruit with health-promoting anthocyanins by expression of select transcription factors. Nature biotechnology 26 (11):1301-1308. doi: 10. 1 038/nbt. 1506
2. Eichenberger M, Hansson A, Fischer D, Durr L, Naesby M (2018) De novo biosynthesis of anthocyanins in Saccharomyces cerevisiae. FEMS yeast research 18 (4). doi:10.1093/femsyr/foy046
3. Levisson M, Patinios C, Hein S, de Groot PA, Daran JM, Hall RD, Martens S, Beekwilder J (2018) Engineering de novo anthocyanin production in Saccharomyces cerevisiae. Microbial cell factories 17 (1):103. doi:10.1186/s12934-018-0951-6
4. Xu S, Li G, Zhou J, Chen G, Shao J (2022) Efficient production of anthocyanins in Saccharomyces cerevisiae by introducing anthocyanin transporter and knocking out endogenous degrading enzymes. Frontiers in bioengineering and biotechnology 10:899182. doi:10.3389/fbioe.2022.899182
5. Houghton A, Appelhagen I, Martin C (2021) Natural Blues: Structure Meets Function in Anthocyanins. Plants (Basel) 10 (4). doi:10.3390/plants10040726
6. Neves MIL, Silva EK, Meireles MAA (2021) Natural blue food colorants: Consumer acceptance, current alternatives, trends, challenges, and future strategies. Trends Food Sci Tech 112:163-173. doi:10.1016/j.tifs.2021.03.023
7. Yoshida K, Mori M, Kondo T (2009) Blue flower color development by anthocyanins: from chemical structure to cell physiology. Nat Prod Rep 26 (7):884-915. doi:10.1039/b800165k
8. Nakatsuka T, Sato K, Takahashi H, Yamamura S, Nishihara M (2008) Cloning and characterization of the UDP-glucose:anthocyanin 5-O-glucosyltransferase gene from blue-flowered gentian. Journal of experimental botany 59 (6):1241-1252. doi:10.1093/jxb/ern031
9. Fukuchi-Mizutani M, Okuhara H, Fukui Y, Nakao M, Katsumoto Y, Yonekura-Sakakibara K, Kusumi T, Hase T, Tanaka Y (2003) Biochemical and molecular characterization of a novel UDP-glucose:anthocyanin 3'-O-glucosyltransferase, a key enzyme for blue anthocyanin biosynthesis, from gentian. Plant physiology 132 (3):1652-1663. doi:10.1104/pp.102.018242
10. Fujiwara H, Tanaka Y, Yonekura-Sakakibara K, Fukuchi-Mizutani M, Nakao M, Fukui Y, Yamaguchi M, Ashikari T, Kusumi T (1998) cDNA cloning, gene expression and subcellular localization of anthocyanin 5-aromatic acyltransferase from Gentiana triflora. The Plant journal : for cell and molecular biology 16 (4):421-431. doi:10.1046/j.1365-313x.1998.00312.x
11. Kogawa K, Kato N, Kazuma K, Noda N, Suzuki M (2007) Purification and characterization of UDP-glucose: anthocyanin 3',5'-O-glucosyltransferase from Clitoria ternatea. Planta 226 (6):1501-1509. doi:10.1007/s00425-007-0584-1
12. Mori M, Kondo T, Yoshida K (2009) Anthocyanin components and mechanism for color development in blue Veronica flowers. Biosci Biotechnol Biochem 73 (10):2329-2331. doi:10.1271/bbb.90349
13. Ono E, Ruike M, Iwashita T, Nomoto K, Fukui Y (2010) Co-pigmentation and flavonoid glycosyltransferases in blue Veronica persica flowers. Phytochemistry 71 (7):726-735. doi:10.1016/j.phytochem.2010.02.008
14. Xia Y, Li K, Li J, Wang T, Gu L, Xun L (2019) T5 exonuclease-dependent assembly offers a low-cost method for efficient cloning and site-directed mutagenesis. Nucleic acids research 47 (3):e15. doi:10.1093/nar/gky1169
15. Grutzner R, Konig K, Horn C, Engler C, Laub A, Vogt T, Marillonnet S (2023) A transient expression tool box for anthocyanin biosynthesis in Nicotiana benthamiana. Plant biotechnology journal. doi:10.1111/pbi.14261
16. Deikman J, Xu R, Kneissl ML, Ciardi JA, Kim KN, Pelah D (1998) Separation of cis elements responsive to ethylene, fruit development, and ripening in the 5'-flanking region of the ripening-related E8 gene. Plant molecular biology 37 (6):1001-1011. doi:10.1023/a:1006091928367
17. Marillonnet S, Grutzner R (2020) Synthetic DNA Assembly Using Golden Gate Cloning and the Hierarchical Modular Cloning Pipeline. Current protocols in molecular biology 130 (1):e115. doi:10.1002/cpmb.115
18. Yang T, Ali M, Lin L, Li P, He H, Zhu Q, Sun C, Wu N, Zhang X, Huang T, Li CB, Li C, Deng L (2023) Recoloring tomato fruit by CRISPR/Cas9-mediated multiplex gene editing. Horticulture research 10 (1):uhac214. doi:10.1093/hr/uhac214
19. Floss DS, Schliemann W, Schmidt J, Strack D, Walter MH (2008) RNA interference-mediated repression of MtCCD1 in mycorrhizal roots of Medicago truncatula causes accumulation of C27 apocarotenoids, shedding light on the functional role of CCD1. Plant physiology 148 (3):1267-1282. doi:10.1104/pp.108.125062

## Claims

1. A process of producing delphinidin 3-O-[2-O-(6-O-p-coumaroyl)-glucosyl-6-O-p-coumaroyl] glucoside comprising the cation of formula (IV) or a neutral form or anionic form thereof from delphinidin 3-O-(2-O-glucosyl-6-O-p-coumaroyl) glucoside comprising the cation of formula (**III**) or a neutral form or anionic form thereof: 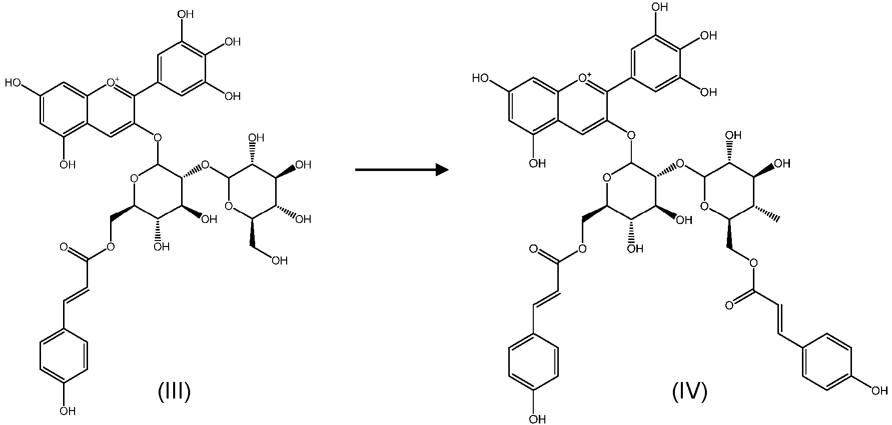 in eukaryotic cells, comprising expressing, in said in eukaryotic cells, an acyltransferase comprising a polypeptide comprising or consisting of:
(a) the amino acid sequence of SEQ ID NO: 3 or
(b) an amino acid sequence having at least 80 % sequence identity to the amino acid sequence of SEQ ID NO: 3 or
(c) an amino acid sequence having from 1 to 40 amino acid residue substitutions, additions, insertions and/or deletions compared to the amino acid sequence of **SEQ ID NO: 3.**

2. A process of producing delphinidin 3-*O*-[2-*O*-(6-*O*-p-coumaroyl-glucosyl)-6-O-p-coumaroyl] glucoside 5-*O*-glucoside comprising the cation of formula **(V)** or a neutral form or anionic form thereof from delphinidin 3-*O*-[2-*O*-(6-*O*-p-coumaroyl)-glucosyl-6-*O*-p-coumaroyl] glucoside comprising the cation of formula **(IV)** or a neutral form or anionic form thereof: 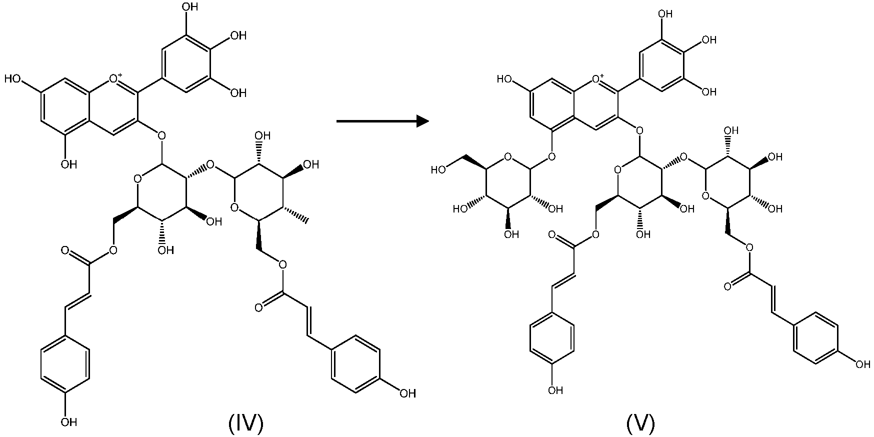 in eukaryotic cells, comprising expressing, in said in eukaryotic cells, a glucosyltransferase comprising a polypeptide comprising or consisting of:
(a) the amino acid sequence of **SEQ ID NO: 4** or
(b) an amino acid sequence having at least 80 % sequence identity to the amino acid sequence of **SEQ ID NO: 4** or
(c) an amino acid sequence having from 1 to 40 amino acid residue substitutions, additions, insertions and/or deletions compared to the amino acid sequence of **SEQ ID NO: 4.**

3. A process of producing delphinidin 3-*O*-(2-*O*-glucosyl-6-*O*-p-coumaroyl) glucoside comprising the cation of formula (**III**) or a neutral form or anionic form thereof from delphinidin 3-*O*-(6-*O*-p-coumaroyl)-glucoside comprising the cation of formula **(II)** or a neutral form or anionic form thereof: 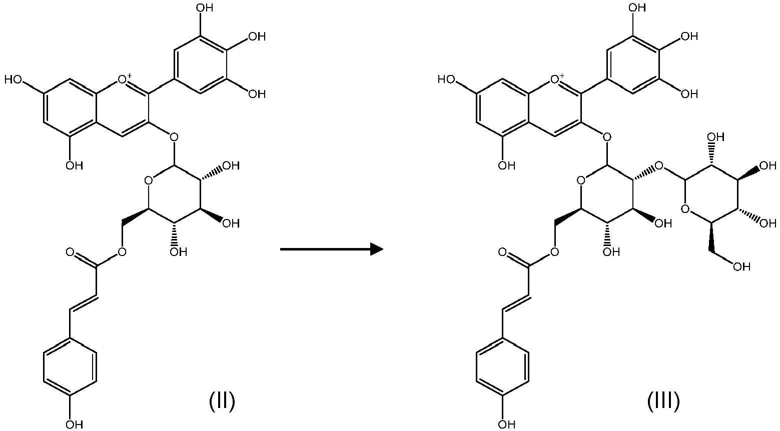 in eukaryotic cells, comprising expressing, in said eukaryotic cells, a glucosyltransferase comprising a polypeptide comprising or consisting of:
(a) the amino acid sequence of **SEQ ID NO: 2** or
(b) an amino acid sequence having at least 80 % sequence identity to the amino acid sequence of **SEQ ID NO: 2** or
(c) an amino acid sequence having from 1 to 40 amino acid residue substitutions, additions, insertions and/or deletions compared to the amino acid sequence of **SEQ ID NO: 2.**

4. A process of producing delphinidin 3-*O*-(6-*O*-p-coumaroyl) glucoside comprising the cation of formula (II) or a neutral form or anionic form thereof from delphinidin 3-*O-*glucoside (D3G) comprising the cation of formula (**I**) or a neutral form or anionic form thereof: 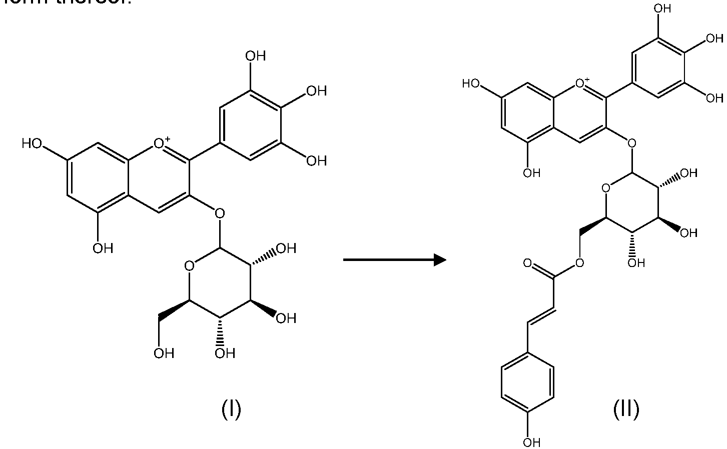 in eukaryotic cells, comprising expressing, in said eukaryotic cells, an acyltransferase comprising a polypeptide comprising or consisting of:
(a) the amino acid sequence of **SEQ ID NO: 1** or
(b) an amino acid sequence having at least 80 % sequence identity to the amino acid sequence of **SEQ ID NO: 1** or
(c) an amino acid sequence having from 1 to 40 amino acid residue substitutions, additions, insertions and/or deletions compared to the amino acid sequence of **SEQ ID NO: 1.**

5. A process of producing delphinidin 3-*O*-[2-*O*-(6-*O*-p-coumaroyl-glucosyl)-6-*O*-p-coumaroyl] glucoside 5-*O*-(6-*O*-malonyl)-glucoside comprising the cation of formula (VI) or a neutral form or anionic form thereof from delphinidin 3-*O*-[2-*O*-(6-*O*-p-coumaroyl-glucosyl)-6-*O*-p-coumaroyl] glucoside 5-O-glucoside comprising the cation of formula (V) or a neutral form or anionic form thereof: 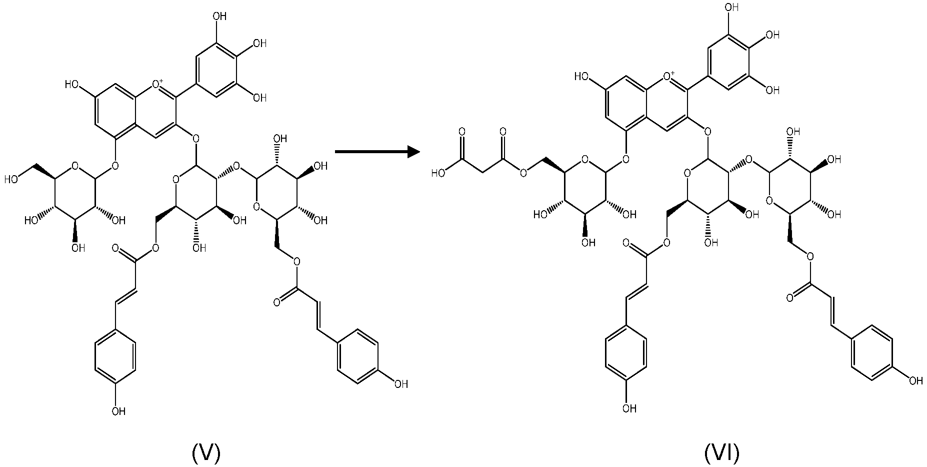 in eukaryotic cells, comprising expressing, in said in eukaryotic cells, a malonyltransferase comprising a polypeptide comprising or consisting of:
(a) the amino acid sequence of **SEQ ID NO: 5** or
(b) an amino acid sequence having at least 80 % sequence identity to the amino acid sequence of **SEQ ID NO: 5** or
(c) an amino acid sequence having from 1 to 40 amino acid residue substitutions, additions, insertions and/or deletions compared to the amino acid sequence of **SEQ ID NO: 5.**

6. A process of producing delphinidin 3-*O*-[2-*O*-(6-*O*-p-coumaroyl)-glucosyl-6-O-p-coumaroyl] glucoside comprising the cation of formula **(IV)** or a neutral form or anionic form thereof from D3G comprising the cation of formula (**I**) or a neutral form or anionic form thereof in eukaryotic cells, comprising expressing, in said eukaryotic cells,
(i) an acyltransferase, preferably as defined in claim 4 (AT1),
(ii) a glucosyltransferase as defined in claim 3 (UGT94F1), and
(iii) an acyltransferase as defined in claim 1 (AT2).

7. A process of producing delphinidin 3-*O*-[2-*O*-(6-*O*-p-coumaroyl-glucosyl)-6-*O*-p-coumaroyl] glucoside 5-O-glucoside comprising the cation of formula **(V)** or a neutral or anionic form thereof from delphinidin-3-*O*-glucoside (D3G) comprising the cation of formula (**I**) or a neutral or anionic form thereof in eukaryotic cells, comprising expressing in said cells
(i) an acyltransferase, preferably as defined in claim 4 (AT1),
(ii) a glucosyltransferase as defined in claim 3 (UGT94F1),
(iii) an acyltransferase as defined in claim 1 (AT2), and
(iv) a glucosyltransferase as defined in claim 2 (5GT).

8. A process of producing the blue anthocyanin delphinidin 3-*O*-[2-*O*-(6-*O*-p-coumaroyl-glucosyl)-6-*O*-p-coumaroyl] glucoside 5-*O*-(6-*O*-malonyl)-glucoside comprising the cation of formula **(VI)** or a neutral or anionic form thereof from delphinidin-3-*O*-glucoside (D3G) comprising the cation of formula (**I**) or a neutral or anionic form thereof in eukaryotic cells, comprising expressing in said cells
(i) an acyltransferase, preferably as defined in claim 4,
(ii) a glucosyltransferase as defined in claim 3,
(iii) an acyltransferase as defined in claim 1, and
(iv) a glucosyltransferase as defined in claim 2, and
(v) a malonyltransferase, preferably as defined in claim 5.

9. The process according to claim 6, 7 or 8, further comprising adding or providing delphinidin-3-O-glucoside (D3G) to said cells or providing genes to said cells allowing production of D3G in said cells.

10. The process according to any one of claims 1 to 9, wherein said cells are plant cells or yeast cells, or said plant cells are cells of a plant.

11. A protein that is an acyltransferase, comprising a polypeptide comprising or consisting of:
(a) the amino acid sequence of **SEQ ID NO: 3** (Vp At2), or
(b) an amino acid sequence having at least 80 % sequence identity to the amino acid sequence of **SEQ ID NO: 3** or
(c) an amino acid sequence having from 1 to 40 amino acid residue substitutions, additions, insertions and/or deletions to the amino acid sequence of **SEQ ID NO: 3.**

12. A protein that is a glycosyltransferase, comprising a polypeptide comprising or consisting of:
(a) the amino acid sequence of **SEQ ID NO: 4** (Vp 5GT), or
(b) an amino acid sequence having at least 80 % sequence identity to the amino acid sequence of **SEQ ID NO: 4** or
(c) an amino acid sequence having from 1 to 40 amino acid residue substitutions, additions, insertions and/or deletions to the amino acid sequence of **SEQ ID NO: 4.**

13. A protein that is a malonyltransferase, comprising a polypeptide comprising or consisting of:
(a) the amino acid sequence of **SEQ ID NO: 5** (Vp MAT), or
(b) an amino acid sequence having at least 80 % sequence identity to the amino acid sequence of **SEQ ID NO: 5** or
(c) an amino acid sequence having from 1 to 40 amino acid residue substitutions, additions, insertions and/or deletions to the amino acid sequence of **SEQ ID NO: 5.**

14. A polynucleotide encoding one or more polypeptide(s) comprising or consisting of the amino acid sequence of any one of **SEQ ID NO: 1, 3, 4,** or **5,** or
an amino acid sequence having at least 80 % sequence identity to the amino acid sequence of any one of **SEQ ID NO: 1, 3, 4,** or **5,** or
an amino acid sequence having from 1 to 40 amino acid residue substitutions, additions, insertions and/or deletions to the amino acid sequence of any one of **SEQ ID NO: 1, 3, 4,** or **5;** or
a polynucleotide comprising or consisting of the nucleotide sequence of **SEQ ID NO: 6, 8, 9,** and/or **10;** or
a vector or recombinant construct comprising the polynucleotide.

15. A plant or plant cell transfected or transformed with a polynucleotide according to claim 14, preferably, comprising a gene encoding the protein as defined in claim 11 (AT2) and a gene encoding the protein as defined in claim 12 (5GT).
